# EUROPÄISCHE PATENTSCHRIFT

(11) **EP 0 628 538 B1**
(45) Veröffentlichungstag und Bekanntmachung des Hinweises auf die Patenterteilung: **22.01.1997**
(21) Anmeldenummer: 94112407.5
(22) Anmeldetag: 10.06.1989
(51) Int. Cl.: C07C 229/22, C07C 271/22, C07C 233/83, C07C 251/08

(54) **Verfahren zur acylierung von Threonin-Derivaten und deren Verwendung zur Herstellung von 1,4-Dihydropyridinen**
Process for the acylation of threonine derivatives and their use in the preparation of 1,4-dihydropyridines
Procédé d'acylation de dérivés de la thréonine et leur utilisation dans la préparation de 1,4-dihydropyridines

(30) Priorität: 24.06.1988 DE 3821334
(43) Veröffentlichungstag der Anmeldung: 14.12.1994
(62) Teilanmeldung aus: 89110528.0
(73) Patentinhaber: BAYER AG, 51368 Leverkusen (DE)
(72) Erfinder: Stoltefuss, Jürgen, Dipl.-Ing., D-42781 Haan (DE); Bechem, Martin, Dr., D-42111 Wuppertal (DE); Gross, Rainer, Prof. Dr., D-42115 Wuppertal (DE); Hebisch, Siegbert, Dr., D-46244 Bottrop (DE); Schramm, Matthias, Dr., D-51375 Leverkusen (DE)

(56) Entgegenhaltungen:
- J. ANTIBIOTICS, Bd.41, Nr.11, November 1988 Seiten 1568 - 1574 TAKAHASHI, ATSUSHI; NAKAMURA, HIKARU; IKEDA, DAISHIRO; NAGANAWA, HIROSHI; KAMEYAMA, TOSHIYUKI; KURASAWA, SHOGO; OKAMI, YOSHIRO; TA 'Thrazarine, a new antitumor antibiotic. II. Physicochemical properties and structure determination'
- BULLETIN OF THE CHEMICAL SOCIETY OF JAPAN., Bd.52, Nr.10, Oktober 1979, TOKYO JP Seiten 3111 - 3112 KINOSHITA, HIDEKI; ISHIKAWA, HAJIME; KOTAKE, HIROSHI 'A synthetic method for N-(benzyloxycarbonyl) hydroxy amino acid t-butyl esters'
- BULLETIN OF THE CHEMICAL SOCIETY OF JAPAN., Bd.52, Nr.10, Oktober 1979, TOKYO, JP; Seiten 3111-3112, "A synthetic method for N-(benzyloxycarbonyl) hydroxy amino acid t-butyl esters"

## Beschreibung

Die vorliegende Teilanmeldung aus der Anmeldung Nr. 89 110 528.0 betrifft neue Zwischenprodukte und Verfahren zu ihrer Herstellung sowie ihre Verwendung bei der Herstellung von 1,4-Dihydropyridinen.

Es ist bereits bekannt geworden, daß man Dihydropyridincarbonsäuren aus den entsprechenden Dihydropyridincarbonsäure-β-cyanoeethylestern darstellen kann (vgl. DOS 39 29 545).

In der Publikation Bull. of the Chem. Soc., Japan, Bd. 52 (10), Seiten 3111-3112 (1979) wird bereits die Umsetzung von Diketen mit einem Carboxylgruppen tragenden Threoninderivat in Gegenwart äquimolarer Mengen Triethylamin beschrieben. Für die nachfolgende Veresterung werden mehrere Tage benötigt und das dort beschriebene Reinigungsverfahren über präparative Dünnschichtchromatographie ist kaum geeignet für ein technisches Herstellungsverfahren, das zumindest im kg-Maßstab arbeiten sollte.

Gegenstand der Erfindung sind Verbindungen der allgemeinen Formeln (II) 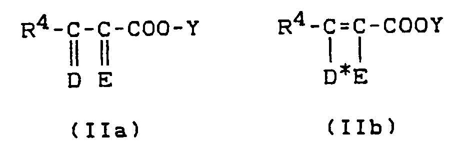 in welcher
- R⁴: für geradkettiges oder verzweigtes Alkyl oder Alkenyl mit bis zu 12 Kohlenstoffatomen steht. das gegebenenfalls substituiert sein kann durch Halogen, Hydroxy, C₁-C₆-Alkoxyl Trifluormethyl, Carboxy, C₁-C₆-Alkoxycarbonyl oder Phenyl, das substituiert sein kann durch Nitro, Halogen, C₁-C₂-Alkyl, C₁-C₄-Alkoxy, Trifluormethyl oder Trifluormethoxy, oder
- für Cycloalkyl mit 3 bis 8 Kohlenstoffatomen steht, oder
- für Phenyl steht, das gegebenenfalls substituiert sein kann durch Nitro, Halogen, C₁-C₂-Alkyl oder C₁-C₄-Alkoxy,
und
- Y -: für einen Rest 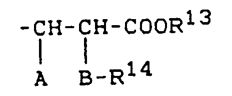 steht;
worin
A - für Wasserstoff oder Methyl steht
und
B - für eine Gruppe der Formel -NH, -NH-CO-, -NH-CS-, -NH-COO-, -NH-SO₂- oder -NH-CO-NH- oder -NH-CS-NH- steht.
R¹³ -
- für geradkettiges oder verzweigtes Alkyl mit bis zu 12 Kohlenstoffatomen steht, das substituiert sein kann durch Halogen, Hydroxy, Carboxy, Cyano, C₁-C₈-Alkoxycarbonyl, Carbonyl, Alkylamino oder Dialkylamino mit bis zu 8 Kohlenstoffatomen, Carbamoyl, C₁-C₆-Alkoxy oder Phenyl, das substituiert sein kann durch Nitro, Cyano, Trifluormethyl, Trifluormethoxy, Halogen, C₁-C₆-Alkoxy oder C₁-C₂-Alkyl, oder
- für Cycloalkyl mit 3 bis 8 Kohlenstoffatomen, oder
- für Phenyl steht, das substituiert sein kann durch Halogen, C₁-C₃-Alkyl, C₁-C₃-Alkoxy, C₁-C₃-Halogenalkyl oder C₁-C₃-Halogenalkoxy,

R¹⁴ - für Wasserstoff steht, oder
- für geradkettiges oder verzweigtes Alkyl oder Alkenyl mit jeweils bis zu 12 Kohlenstoffatomen steht, das substituiert sein kann durch Halogen, Hydroxy, C₁-C₈-Alkoxy, Nitro, Cyano, C₁-C₈-Alkylthio, Carboxy, C₁-C₈-Alkoxycarbonyl oder Phenyl, das substutiert sein kann durch Nitro, Cyano, Trifluormethyl, Trifluormethoxy, C₁-C₈-Alkyl, Halogen oder C₁-C₈-Alkoxy, oder
- für Cycloalkyl mit 3 bis 8 Kohlenstoffatomen steht; oder
- für Aryl mit 6 bis 10 Kohlenstoffatomen steht, das bis zu 5-fach gleich oder verschieden substituiert sein kann durch Nitro; Cyano, Halogen, C₁-C₈-Alkyl, C₁-C₈-Alkoxy, C₁-C₈-Alkylthio, Carbamoyl, Dialkylcarbamoyl mit jeweils bis zu 6 Kohlenstoffatomen je Alkylgruppe, Carboxy, C₁-C₈-Alkoxycarbonyl, C₁-C₆-Halogenalkyl, C₁-C₆-Halogenalkoxy, C₁-C₆-Halogenalkylthio, C₁-C₆-Alkylsulfonyl, C₁-C₆-Alkylsulfamoyl, Amino, C₁-C₈-Alkylamino, Di-alkylamino mit jeweils bis zu 8 Kohlenstoffatomen je Alkylgruppe oder C₁-C₈-Acylamino; oder
- für einen 5- bis 7-gliedrigen gesättigten oder ungesättigten, heterocyclischen Ring steht, der als Heteroatom ein bis drei Sauerstoff-, Schwefel- und/oder Stickstoffatome enthalten kann,

D - für Sauerstoff oder für die Gruppe =NH steht und
D*- für Hydroxy oder die Gruppe NH₂ steht, und
E - für Wasserstoff oder im Falle (IIa) für Wasserstoff oder die Gruppe =CHR¹ steht, wobei
R¹- für geradkettiges oder verzweigtes Alkyl mit bis zu 6 Kohlenstoffatomen steht, das gegebenenfalls substituiert sein kann durch Halogen, Hydroxy, C₁-C₈-Alkoxy, Carboxy, C₁-C₈-Alkoxycarbonyl oder Phenyl, oder
- für Cycloalkyl mit 3 bis 6 Kohlenstoffatomen steht, oder
- für Aryl mit 6 bis 10 Kohlenstoffatomen steht, das bis zu 3-fach gleich oder verschieden substituiert sein kann durch Nitro, Cyano, C₁-C₆-Halogenalkyl, Halogen, C₁-C₆-Alkyl, C₁-C₆-Alkoxycarbonyl oder durch C₁-C₆-Halogenalkoxy, C₁-C₄-Halogenalkylthio, Carbamoyl, Dialkylcarbamoyl mit bis zu 6 Kohlenstoffatomen je Alkylgruppe, C₂-C₈-Alkenyl, das gegebenenfalls substituiert sein kann durch C₁-C₆-Alkoxycarbonyl, oder durch gegebenenfalls durch C₁-C₆-Alkyl, C₁-C₆-Alkoxy, Nitro, Halogen, Cyano, C₁-C₄-Halogenalkyl oder C₁-C₄-Halogenalkoxy substituiertes Phenylsulfonyloxy, oder durch C₁-C₈-Alkylamino, Dialkylamino mit jeweils bis zu 6 Kohlenstoffatomen je Alkylgruppe, C₁-C₈-Acylamino oder durch C₁-C₈-Alkoxy oder C₁-C₈-Alkylthio, die gegebenenfalls substituiert sein können durch Cyclohexyl oder Phenyl, das seinerseits substituiert sein kann durch Halogen, C₁-C₆-Alkyl, C₁-C₆-Alkoxy, Nitro, C₁-C₄-Halogenalkyl, C₁-C₂-Alkoxycarbonyl, Cyano oder C₁-C₄-Halogenalkoxy, oder
- für einen mono-, bi- oder tricyclischen, gesättigten oder ungesättigten Heterocyclus steht, der bis zu 3 Heteroatome aus der Gruppe H, O oder S enthalten kann, der substituiert sein kann durch C₁-C₄-Alkylthio,
mit der Maßgabe, daß Y nicht tert.-Butyl sein kann, wenn R⁴ für Methyl, D für Sauerstoff und E für zwei Wasserstoffatome steht.

Die erfindungsgemäßen Verbindungen existieren in stereoisomeren Formen, die sich entweder wie Bild und Spiegelbild (Enantiomere) oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen (vgl. E.L. Eliel, Stereochemistry of Carbon Compounds, McGraw Hill, 1962).

Bevorzugt sind Verbindungen der allgemeinen Formel (II),
in welcher
- R¹ -: für geradkettiges oder verzweigtes Alkyl mit bis zu 4 Kohlenstoffatomen steht, das gegebenenfalls substituiert sein kann durch Phenyl, C₁-C₂-Alkoxycarbonyl oder Carboxy, oder
- für Phenyl steht, das bis zu 3-fach gleich oder verschieden substituiert sein kann durch Nitro, Cyano, C₁-C₄-Halogenalkyl, Halogen, C₁-C₄-Alkyl, C₁-C₄-Alkoxycarbonyl, C₁-C₂-Halogenalkoxy, C₁-C₂-Halogenalkylthio, C₁-C₄-Alkoxycarbonyl, oder durch gegebenenfalls durch C₁-C₂-Alkyl oder Halogen, substituiertes Phenylsulfonyloxy oder durch C₁-C₄-Alkoxy oder C₁-C₄-Alkylthio, die substituiert sein können durch Cyclohexyl oder Phenyl, das gegebenenfalls substituiert sein kann durch Halogen, C₁-C₄-Alkyl, C₁-C₄-Alkoxy, Nitro, C₁-C₄-Halogenalkyl, Cyano oder C₁-C₄-Halogenalkoxy, oder
- für einen mono- oder bicyclischen ungesättigten Heterocyclus steht, der bis zu 3 Heteroatome aus der Gruppe H, O oder S enthalten kann, der gegebenenfalls substituiert sein kann durch C₁-C₃-Alkylthio, und
- R⁴: - für geradkettiges oder verzweigtes Alkyl mit bis zu 6 Kohlenstoffatomen steht, das durch Halogen, Hydroxy, Carboxy, C₁-C₄-Alkoxycarbonyl substituiert sein kann, oder durch Phenyl substituiert sein kann, das seinerseits substituiert sein kann durch Halogen, C₁-C₂-Alkyl, C₁-C₂-Alkoxy, Trifluormethyl oder Trifluormethoxy, oder
- für Cycloalkyl mit 3 bis 6 Kohlenstoffatomen steht, oder
- für Phenyl steht, das gegebenenfalls substituiert sein kann durch Halogen, C₁-C₂-Alkyl oder C₁-C₂-Alkoxy
und
- Y -: für einen Rest 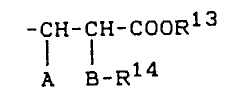 steht, worin
A - für Wasserstoff oder Methyl steht
und
B - für eine Gruppe der Formel -NH, -NH-CO-, -NH-COO-, -NH-SO₂- oder -NH-CO-NH- steht
und
R¹³
- für geradkettiges oder verzweigtes Alkyl mit bis zu 8 Kohlenstoffatomen steht, das substituiert sein kann durch Halogen, Hydroxy, C₁-C₃-Alkoxy, Trifluormethyl, Carboxy, C₁-C₆-Alkoxycarbonyl oder Phenyl, das seinerseits substituiert sein kann durch Trifluormethyl, Trifluormethoxy, Halogen, C₁-C₃-Alkoxy oder C₁-C₂-Alkyl, oder
- für Cycloalkyl mit 3 bis 6 Kohlenstoffatomen steht, oder
- für Phenyl steht, das durch Halogen, C₁-C₂-Alkyl oder C₁-C₂-Alkoxy substituiert sein kann,
und
R¹⁴ -
- Wasserstoff bedeutet, oder
- für geradkettiges oder verzweigtes Alkyl oder Alkenyl mit jeweils bis zu 8 Kohlenstoffatomen steht, das substituiert sein kann durch Halogen, Hydroxy, C₁-C₄-Alkoxy, Cyano, C₁-C₄-Alkylthio, Carboxy, C₁-C₄-Alkoxycarbonyl oder Phenyl, das seinerseits substituiert sein kann durch Trifluormethyl, Trifluormethoxy, C₁-C₄-Alkyl, Halogen oder C₁-C₄-Alkoxy, oder
- für Cycloalkyl mit 3 bis 6 Kohlenstoffatomen steht, oder
- für Phenyl steht, das bis zu 2-fach gleich oder verschieden substituiert sein kann durch Nitro, Cyano, Halogen, C₁-C₄-Alkyl, C₁-C₄-Alkoxy, C₁-C₄-Alkylthio, Carbamoyl, Dialkylcarbamoyl mit bis zu 4 Kohlenstoffatomen je Alkylgruppe, Carboxy, C₁-C₆-Alkoxycarbonyl, C₁-C₃-Halogenalkyl, C₁-C₃-Halogenalkoxy, C₁-C₃-Halogenalkylthio, C₁-C₃-Alkylsulfonyl, C₁-C₃-Alkylsulfamoyl, Amino, C₁-C₄-Alkylamino, Dialkylamino mit jeweils bis zu 4 Kohlenstoffatomen je Alkylgruppe oder C₁-C₄-Acylamino, oder
- für einen 5- bis 6-gliedrigen ungesättigten heterocyclischen Ring steht, der als Heteroatom ein Stickstoffatom enthalten kann.

Besonders bevorzugt sind Verbindungen der allgemeinen Formel (II),
in welcher
- R¹ -: für geradkettiges oder verzweigtes Alkyl mit bis zu 4 Kohlenstoffatomen steht, oder
- für Cyclopropyl, Cyclopentyl oder Cyclohexyl steht, oder
- für Phenyl steht, das bis zu 2-fach gleich oder verschieden substituiert sein kann durch Nitro, Cyano, Trifluormethyl, Fluor, Chlor, Methyl, Methoxy, Trifluormethoxy, Difluormethoxy, gegebenenfalls durch Methyl, Fluor oder Chlor substituiertes Phenylsulfonyloxy, oder durch C₁-C₄-Alkoxy oder C₁-C₄-Alkylthio, die jeweils substituiert sein können durch Cyclohexyl oder Phenyl, das seinerseits durch Methyl, Fluor, Chlor oder Methoxy substituiert sein kann, oder - für Pyridyl steht
und
- R⁴ -: für geradkettiges oder verzweigtes Alkyl mit bis zu 4 Kohlenstoffatomen steht, oder
- für Cyclopropyl steht,
und
- Y -: für einen Rest 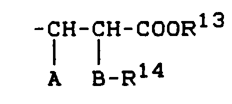 steht, worin
A - für Wasserstoff oder Methyl steht,
und
B - für eine Gruppe der Formel -NH-, -NH-CO-, -NH-COO-, -NH-SO₂- oder -NH-CO-NH- steht,
und
R¹³ - für geradkettiges oder verzweigtes Alkyl mit bis zu 6 Kohlenstoffatomen steht, das substituiert sein kann durch Hydroxy, Methoxy oder Phenyl,
und
R¹⁴
- Wasserstoff bedeutet, oder
- für geradkettiges oder verzweigtes Alkyl mit bis zu 6 Kohlenstoffatomen steht, das substituiert sein kann durch Hydroxy, C₁-C₂-Alkoxycarbonyl oder durch Phenyl, das seinerseits substituiert sein kann durch Methyl, Trifluormethyl, Fluor oder Chlor, oder
- für Cyclopropyl, Cyclopentyl oder Cyclohexyl steht, oder
- für Phenyl steht, das bis zu 2-fach gleich oder verschieden substituiert sein kann durch Nitro, Cyano, Fluor, Chlor, C₁-C₂-Alkyl, C₁-C₄-Alkoxy, C₁-C₂-Alkylthio, Carbamoyl, Carboxy, C₁-C₄-Alkoxycarbonyl, Trifluormethyl, Trifluormethoxy, C₁-C₂-Alkylsulfamoyl oder C₁-C₂-Alkylamino, oder
- für Pyridyl steht.

Die erfindungsgemäßen Verbindungen sind neu und können als Zwischenprodukte für die Herstellung von 1,4-Dihydropyridinen mit wertvollen pharmakologischen Eigenschaften eingesetzt werden. Diese Dihydropyridine beeinflussen die Kontraktionskraft des Herzens und können deshalb zur Bekämpfung von cardiovaskulären Erkrankungen verwendet werden. Durch die Möglichkeit, reine Enantiomere herzustellen, lassen sich die gewünschten pharmakologischen Eigenschaften der Verbindungen in unterschiedlichen Richtungen optimieren. Ausführlichere Angaben zur Verwendung der Verbindungen II als Zwischenprodukte sind aus der Stammanmeldung Nr. 89 110 528.0 ersichtlich.

Die Verbindungen der allgemeinen Formel (II) sind neu. Nur L-3-Oxobutansäure-(2-benzyloxycarbonyl-2-tert.butoxy-carbonyl-1-methyl)-ethylester wird als Schutzgruppe beim Aufbau von Aminosäuren eingesetzt [vgl. Bull. Chem. Soc. Jpn. 52 (16), 3111 - 3112].

Gegenstand der Erfindung sind die Ausgangsprodukte der allgemeinen Formel (II), in der R⁴, Y, D und E die oben angegebene Bedeutung haben, mit der Maßgabe, daß Y nicht tert.Butyl sein kann, wenn R⁴ = Methyl, D = Sauerstoff und E = zwei Wasserstoffatome bedeuten.

Die neuen Verbindungen der Formel (II) können nach verschiedenen Verfahren hergestellt werden. So lassen sich z.B. Verbindungen der Formel II, in der
- R⁴ -: für Methyl,
- D -: für Sauerstoff
- E -: für Wasserstoff steht,
und
Y die oben angegebene Bedeutung hat,
herstellen, indem man
[A] Verbindungen der allgemeinen Formel (VIII)

   HO-Y (VIII)

   in welcher
   Y die oben angegebene Bedeutung hat,
   mit Diketen der allgemeinen Formel (IX) 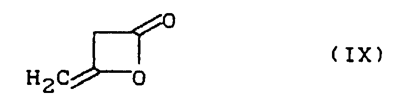 umsetzt.
   Die Verbindungen der Formel (II),
   in der
   R⁴ und Y die oben angegebene Bedeutung haben,
   D - für Sauerstoff
   und
   E - für zwei Wasserstoffatome steht,
   können auch hergestellt werden, indem man
[B] Verbindungen der Formel (VIII) mit Verbindungen der allgemeinen Formel (X) 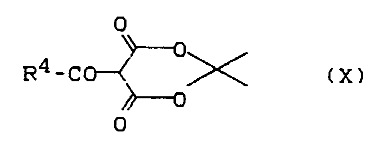 in welcher
   R⁴ die oben angegebene Bedeutung hat,
   umsetzt.
   Man erhält die Verbindungen der allgemeinen Formel (II),
   in der
   R⁴ und Y die oben angegebene Bedeutung haben,
   D - für =NH steht bzw. D* für NH₂ steht,
   und
   E - für Wasserstoff steht,
   indem man
[C] Verbindungen der allgemeinen Formel (II),
   in welcher
   R⁴ und Y die oben angegebene Bedeutung haben,
   D - für Sauerstoff steht
   und
   E - für Wasserstoff steht,
   mit Ammoniak umsetzt.
   Verbindungen der allgemeinen Formel (II),
   in welcher
   R⁴ und Y die oben angegebene Bedeutung haben,
   D - Sauerstoff bedeutet
   und
   E - für die Gruppe R¹-CH= steht,
   erhält man, indem man
[D] Verbindungen der allgemeinen Formel (III),
   in der R¹ die oben angegebene Bedeutung hat,
   mit Verbindungen der allgemeinen Formel (II),
   in welcher
   R⁴ und Y die oben angegebene Bedeutung haben,
   D - für Sauerstoff
   und
   E - für Wasserstoff steht,
   umsetzt.

Die als Ausgangsstoffe eingesetzten Aldehyde der allgemeinen Formel (III) sind bekannt oder können nach bekannten Methoden hergestellt werden [DOS 21 65 260; 24 01 665; T.D. Harris, G.P. Roth, J. Org. Chem. 44, 2004 (1979); W.J. Dale, H.E. Hennis, J. Am. Chem. Soc. 78, 2543 (1956); Chem. Abstr. 59, 13929 (1963)].

Die als Ausgangsstoffe eingesetzten Enamine der allgemeinen Formel (IV) sind bekannt oder können nach bekannten Methoden hergestellt werden [DOS 2 228 377; F.A., Glickman, A.C. Cope, J. Am. Chem. Soc. 67, 1017 (1945)].

Die als Ausgangsstoffe eingesetzten Yliden-β-ketocarbonsäurederivate der allgemeinen Formel (VI) sind bekannt oder können nach bekannten Methoden hergestellt werden [G. Jones "The Knoevenagel Condensation" in Organic Reactions Bd. XV, 204 (1967)].

Die Ausgangsstoffe der Formel (VIII) sind teilweise bekannt oder können nach bekannten Methoden hergestellt werden [vgl. Houben-Weyl, Methoden der organischen Chemie, Band 15, Tei 1, E. Wünsch, Seite 46 ff.].

Die Verbindungen der Formel (IX) und ihre Reaktionen sind bekannt [vgl. R.J. Clemens, Chem. Rev. 88, 241 (1986)].

Die Verbindungen der Formel (X) sind zum Teil bekannt oder können nach üblicher Methode hergestellt werden [vgl. J. Org. Chem. 43, 1087 (1978)].

### Ausführungsbeispiele

### Zwischenprodukt Nr. 1

### Acetessigsäure-(1R, 2S)-(2-benzyloxycarbamoyl-2-methoxycarbonyl-1-methyl)-ethylester

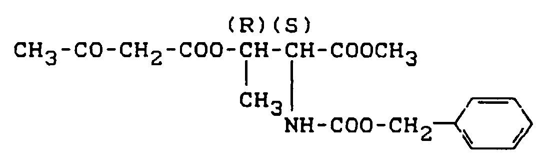

a) Herstellung von (2S, 3R)-2-Amino-3-hydroxybuttersäure-methylester
   109 ml (1,5 mol) Thionylchlorid werden bei 0°C unter starker Kühlung in 240 ml Methanol getropft. Anschließend werden 60 g (0,5 mol) L-Threonin zugefügt und 2 Tage bei Raumtemperatur gerührt, wobei eine klare Lösung entsteht. Nach dem Einengen erhält man 85 g eines farblosen Öles.
b) Herstellung von (2S, 3R)-2-Benzyloxycarbonylamino-3-hydroxybuttersäuremethylester
   34,1 g (0,201 mol) (2S, 3R)-2-Amino-3-hydroxybuttersäuremethylester werden in 300 ml Essigester mit einer Lösung von 40 g Kaliumcarbonat in 200 ml Wasser vermischt. Bei 0°C werden dann 33 ml (0,215 mol) 90 %iges Benzyloxycarbonylchlorid zugetropft. Es wird 2 Stunden bei 0 - 5°C nachgerührt, getrennt, die Essigesterphase 2-mal mit Wasser gewaschen, getrocknet und eingeengt. Die erhaltenen Kristalle werden mit Hexan versetzt und abgesaugt. Man erhält 42,2 g (78,6 %) farblose Kristalle vom Schmelzpunkt 96°C.
   35 g (0,13 mol) (2S, 3R)-2-Benzyloxycarbonylamino-3-hydroxy-buttersäuremethylester werden in 75 ml absolutem Toluol mit 0,2 ml Triethylamin versetzt. Bei 90°C werden 9,8 ml (0,13 mmol) Diketen gelöst in 10 ml absolutem Toluol zugetropft, wobei die Temperatur zwischen 90°C und 98°C gehalten wird. Es wird 2 Stunden bei 95°C nachgerührt, abgekühlt, 1-mal mit Wasser geschüttelt, getrocknet und eingeengt. Man erhält 44,3 g (96,6 % der Theorie) eines braun gefärbten Öles.

### Zwischenprodukt Nr. 2

3-Imino-acetessigsäure-(1R, 2S)-[1-methyl-2-methoxycarbonyl-2-(4-nitrophenylsulfamoyl)]-ethylester bzw. β-Aminocrotonsäure-(1R, 2S)-[1-methyl-2-methoxycarbonyl-2-(4-nitrophenylsulfamoyl)]-ethylester 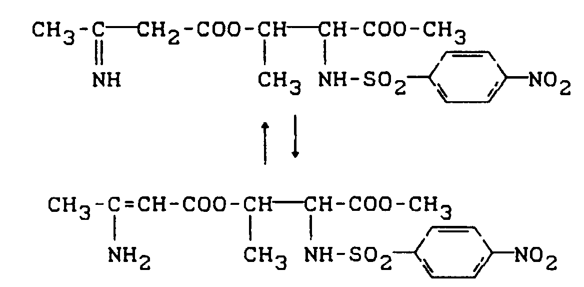 39,2 g (97,5 mmol) Acetessigsäure-(1R, 2S)-[1-methyl-2-methoxycarbonyl-2-(4-nitrophenylsulfamoyl)]-ethylester werden in 370 ml Toluol mit 200 mg p-Toluolsulfonsäure versetzt und 4 Stunden am Wasserabscheider gekocht; wobei gleichzeitig Ammoniak eingeleitet wird. Es wird abgekühlt, 2-mal mit Wasser geschüttelt, getrocknet und eingeengt. Die erhaltenen Kristalle werden mit wenig Ethanol verrührt, abgesaugt und mit Ethanol und Ether gewaschen. Man erhält 20,2 g beigefarbener Kristalle vom Schmelzpunkt 155 - 156°C.

### Zwischenprodukt Nr. 3

2-(2-Benzyloxybenzyliden)-3-oxo-buttersäure-(1R, 2S)-[1-methyl-2-methoxycarbonyl-2-(3,5-dinitrophenylcarbamoyl)]-ethylester 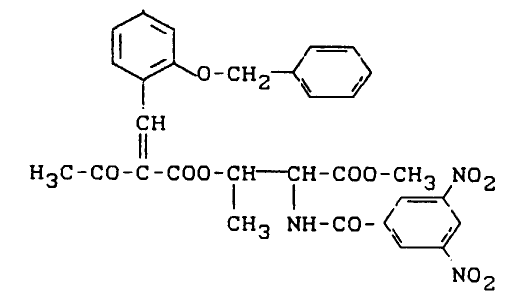 100 g (243,5 mmol) Acetessigsäure-(1R; 2S)-[1-methyl-2-methoxycarbonyl-2-(3,5-dinitrophenylcarbamoyl)]-ethylester werden in 300 ml Isopropanol mit 51,3 g (243,5 mmol) 2-Benzyloxybenzaldehyd verrührt. In diese Suspension wird eine frisch zubereitete Lösung von 1,5 ml Piperidin und 0,75 ml Essigsäure in 12 ml Isopropanol gegeben. Es wird 4 Stunden bei 40 - 45°C gerührt, wobei zuerst ein nicht kristalliner Brei entsteht und dann Kristallisation eintritt. Es wird abgesaugt und mit Isopropanol und Ether gewaschen. Man erhält 123,6 g (83,9 % der Theorie) eines hellbraunen Produktes vom Schmelzpunkt 143 - 146°C.

Analog der Vorschrift der Zwischenproduktes Nr. 1 bis 3 wurden die in der folgenden Tabelle aufgeführten Zwischenprodukte erhalten.

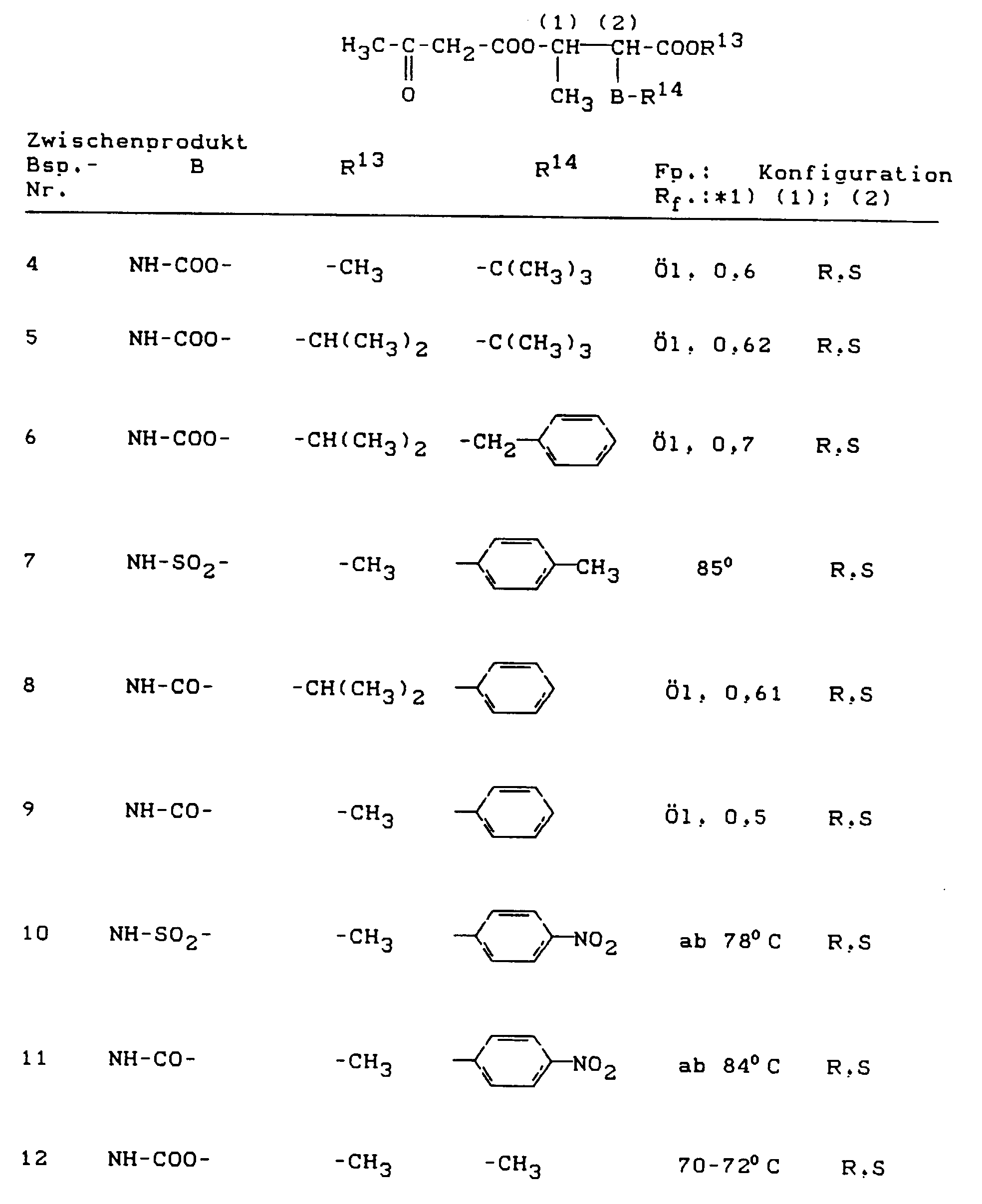

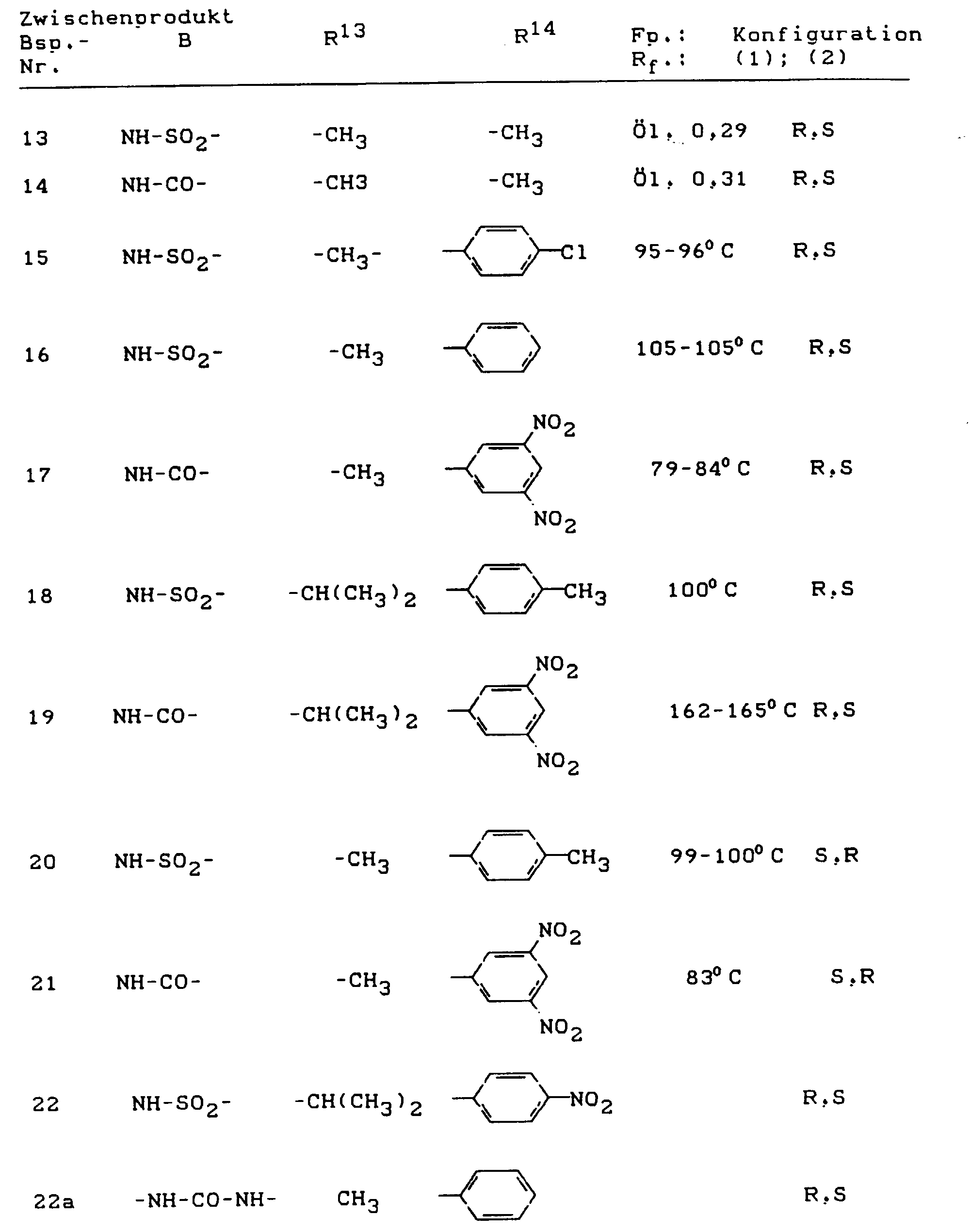

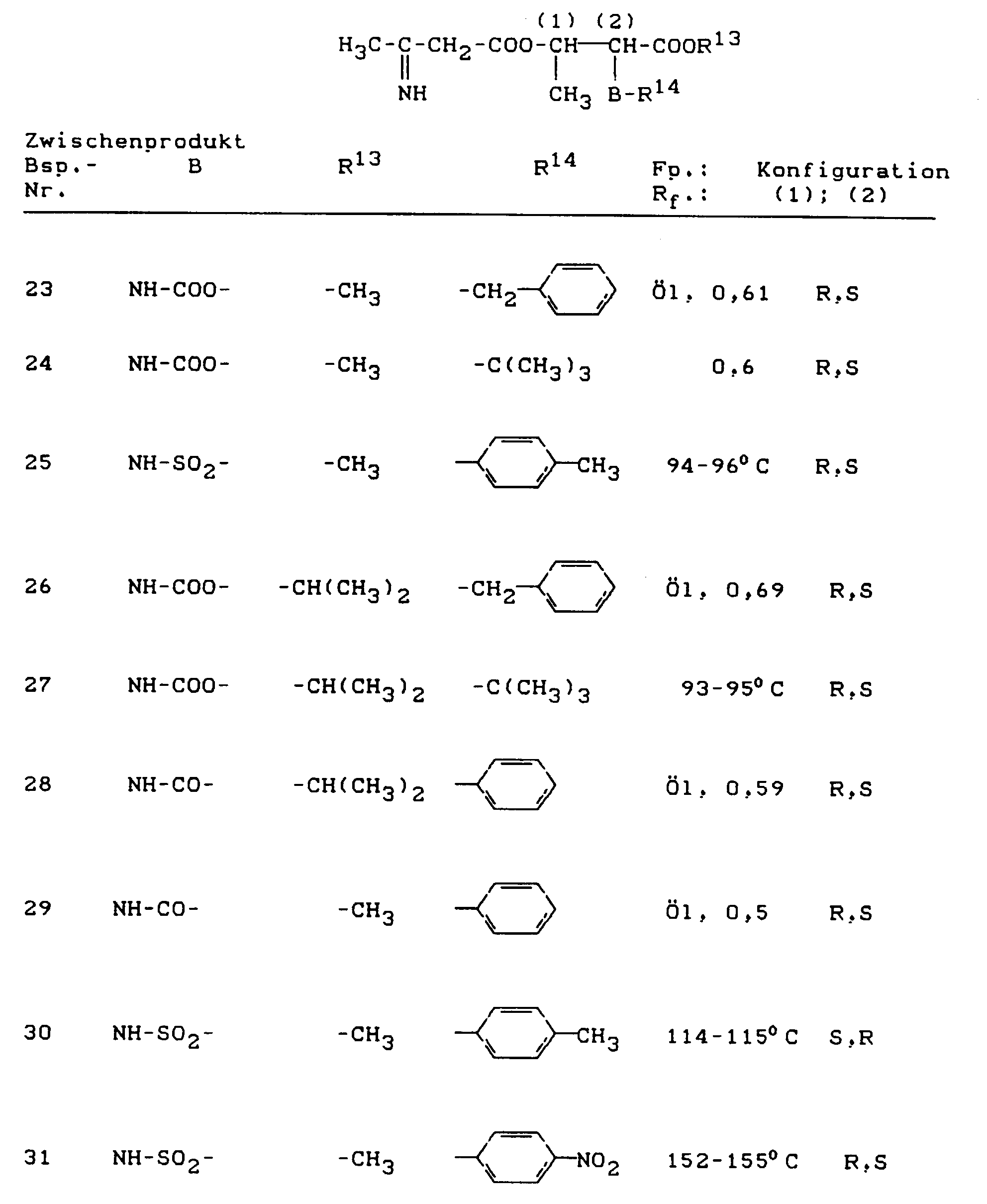

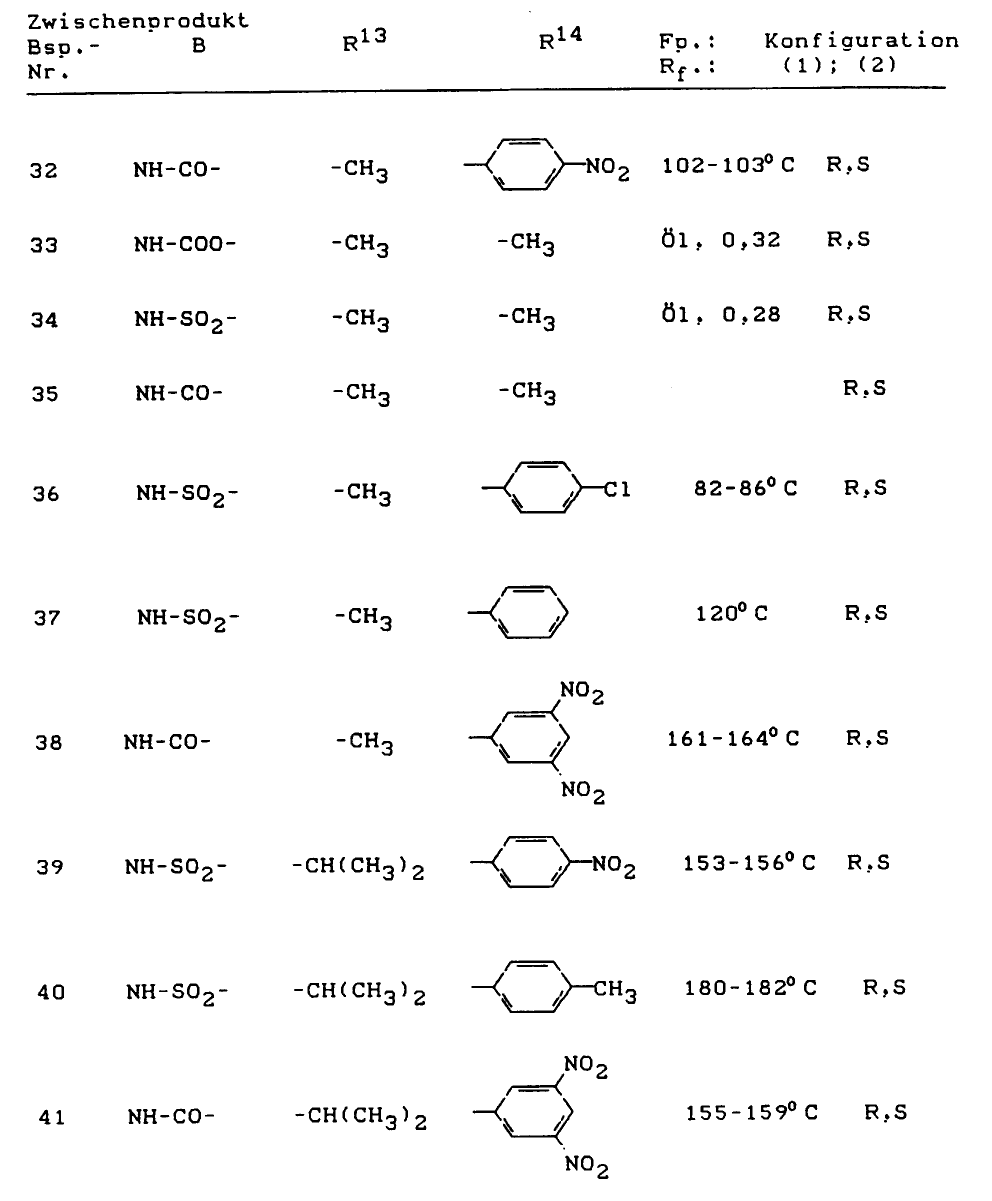

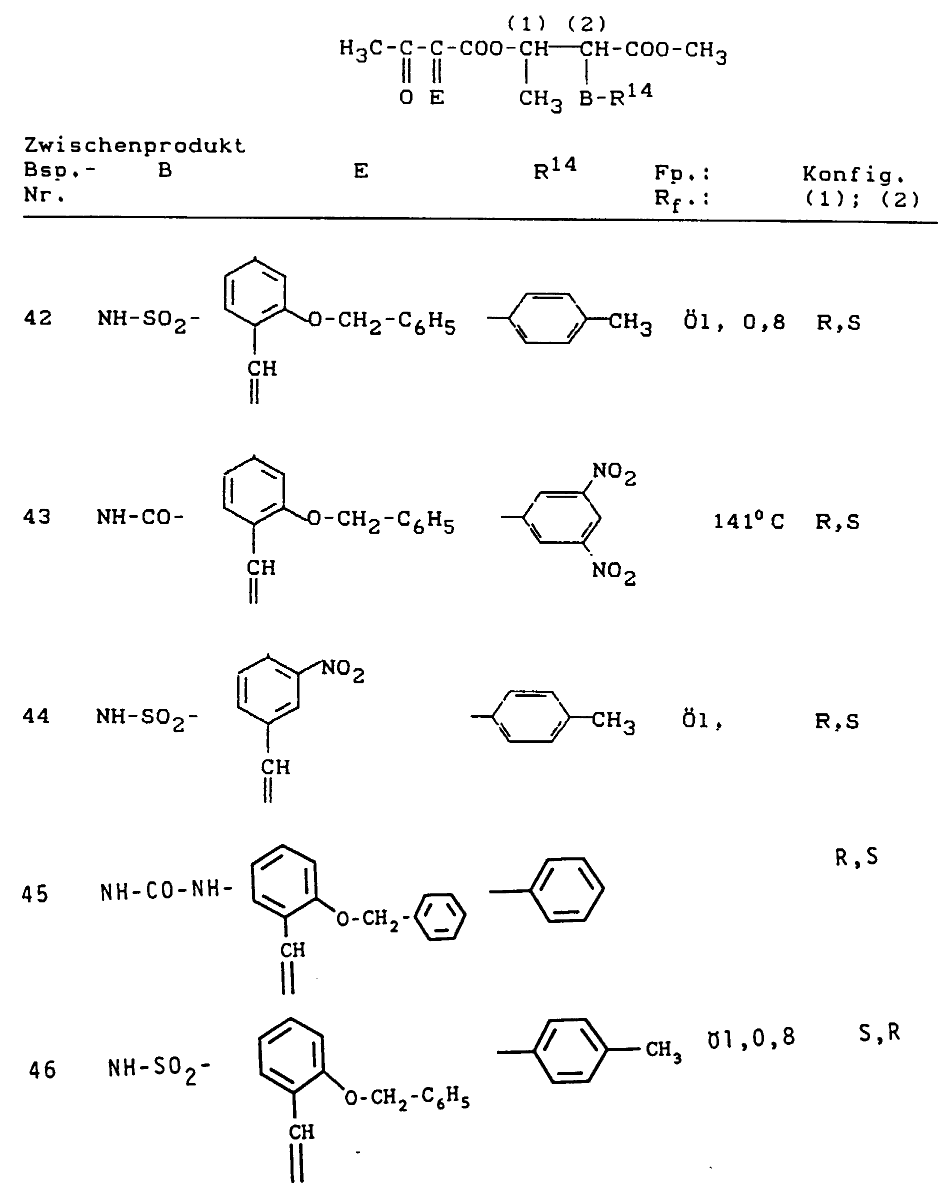

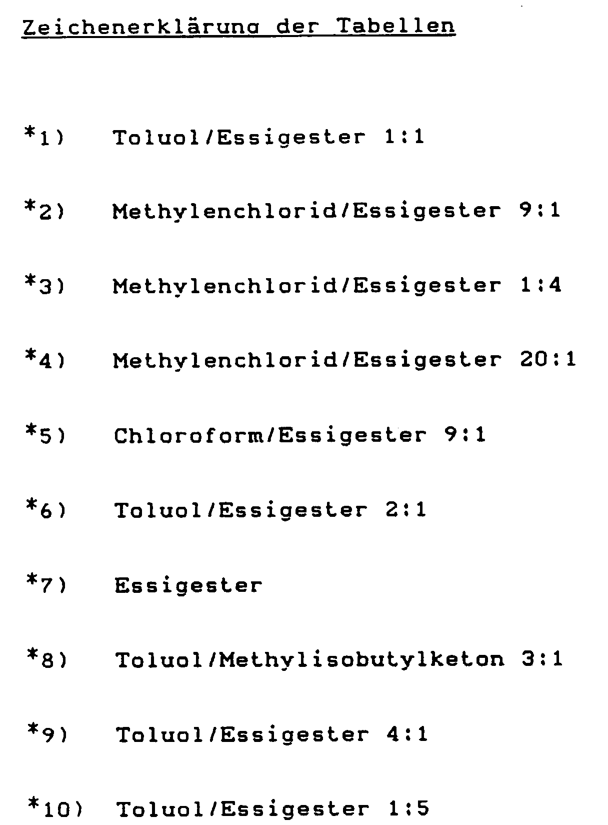

### Verwendungsbeispiele

### Beispiel 1 (mit Diastereomerentrennung)

1,4-Dihydro-2,6-dimethyl-3-nitro-4-phenyl-pyridin-5-carbonsäure-(1R, 2S)-[1-methyl-2-methoxycarbonyl-2-(4-tolyl-sulfamoyl)]-ethylester 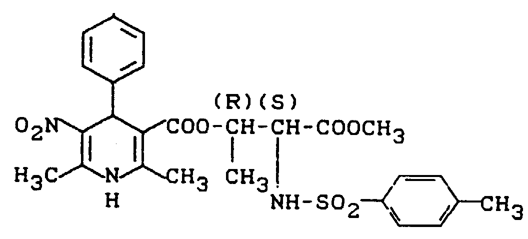 725 mg (3,8 mmol) Benzylidennitroaceton werden in 7 ml Ethanol mit 1,4 g (3,8 mmol) β-Aminocrotonsäure-(1R, 2S)-[1-methyl-2-methoxycarbonyl-2-(4-tolylsulfamoyl)]-ethylester 3 Stunden gekocht. Es wird eingeengt und die beiden Diastereomeren werden durch Säulenchromatographie getrennt. Man erhält 0,6 g eines gelbgefärbten Diastereomeren
R_{f}-Wert = 0,18 und 0,4 g eines Diastereomeren
R_{f}-Wert = 0,146
DC: Kieselgel Alurolle, Kieselgel 60, F 254,
Schichtdicke 0,2 mm (Merck)

- Fließmittel:: Methylenchlorid/Essigester im Volumenverhältnis 20:1

### Beispiel 2 (mit Diastereomerentrennung)

1,4-Dihydro-2,6-dimethyl-4-(3-nitrophenyl)-pyridin-3-carbonsäuremethylester-5-carbonsäure-(1R, 2S)-[1-methyl-2-methoxycarbonyl-2-(4-tolylsulfamoyl)]-ethylester 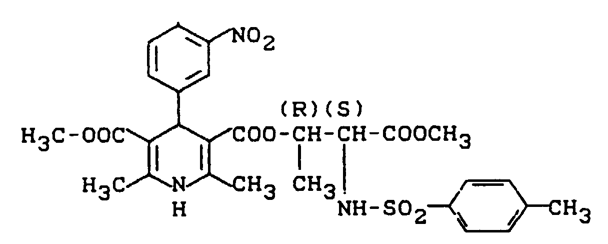 5 g (20 mmol) 2-(3-Nitrobenzyliden)-3-oxo-buttersäuremethylester werden in 40 ml Isopropanol mit 7,4 g (20 mmol) β-Aminocrotonsäure-(1R, 2S)-[1-methyl-2-methoxy-2-(4-tolylsulfamoyl)]-ethylester 5 Stunden gekocht. Es wird abgekühlt, die ausgefallenen Kristalle werden abgesaugt und mit Isopropanol gewaschen. Man erhält 4,8 g (39,9 % der Theorie) eines reinen Diastereomers vom Schmelzpunkt: 157 - 159°C (siehe Beispiel 4).
[α]$\frac{\text{20}}{\text{589}}$ = +49,3° (C = 1,0:DMF).

### Beispiel 3

1,4-Dihydro-2,6-dimethyl-3-nitro-4-phenyl-pyridin-5-carbonsäure-(1R, 2S)-(1-methyl-2-methoxycarbonyl-2-phenyl-carbamoyl)-ethylester 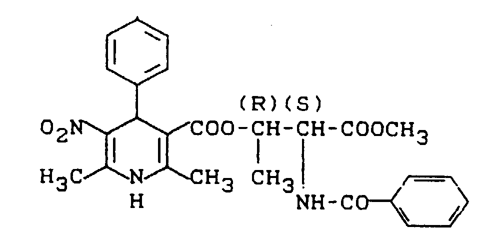 0,37 g (1,95 mmol) Benzyliden-nitroaceton werden mit 0,6 g (1,95 mmol) β-Aminocrotonsäure-(1R, 2S)-(1-methyl-2-methoxycarbonyl-2-phenyl-carbamoyl)-ethylester in 4 ml Ethanol 3 Stunden gekocht. Es wird eingeengt und das Diastereomerengemisch über eine Kieselgelsäule mit Toluol/Essigester gereinigt. Man erhält 0,7 g eines rotgelben Öls vom R_{f}-Wert 0,1 (Fließmittel: Methylenchlorid/Essigester 20:1).

### Beispiel 4 (mit Diastereomerentrennung)

1,4-Dihydro-2,6-dimethyl-4-(3-nitrophenyl)-pyridin-3-carbonsäuremethylester-5-carbonsäure-(1R, 2S)-1-methyl-2-methoxy-carbonyl-2-(p-tolylsulfamoyl)-ethylester Aus der Mutterlauge der Verbindung aus Beispiel 2 werden

3,6 g (29,9 % der Theorie) des zweiten Diastereomers durch Säulenchromatographie als Öl erhalten. R_{f}-Wert: 0,38
Fließmittel: Toluol/Aceton (4:1)

### Beispiel 5 (enantiomere Säure)

(-)1,4-Dihydro-2,6-dimethyl-4-(3-nitrophenyl)-pyridin-3,5-dicarbonsäure-3-methylester 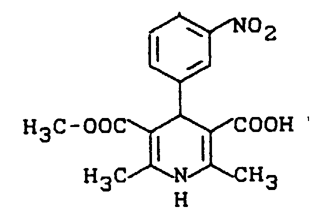 2 g (3,33 mmol) der Verbindung aus Beispiel 2 werden in 18 ml Methanol mit 1,22 ml (8,15 mmol) Diaza-bicycloundecan versetzt und 3 Stunden bei Raumtemperatur gerührt. Es wird eingeengt, der Eindampfrückstand wird mit Wasser versetzt und unter Rühren tropfenweise mit 10 %iger Salzsäure sauer gestellt. Das ausgefallene Festprodukt wird abgesaugt, mit Wasser und anschließend mit Ether gewaschen. Man erhält 1,02 g (88,5% der Theorie) eines farblosen Produktes vom Schmelzpunkt 181 - 182°C unter Zersetzung.
[α]$\frac{\text{20}}{\text{584}}$ = -21 (c = 0,711, Aceton) (Enantiomerenüberschuß (ee) >99 %)
Analog den Beispiel 1 bis 4 wurden die in der folgenden Tabelle aufgeführen Beispiele erhalten.

## Patentansprüche

1. Verbindungen der allgemeinen Formeln (II) 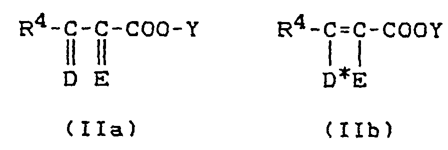 in welcher
R⁴ für geradkettiges oder verzweigtes Alkyl oder Alkenyl mit bis zu 12 Kohlenstoffatomen steht, das gegebenenfalls substituiert sein kann durch Halogen, Hydroxy, C₁-C₆-Alkoxy, Trifluormethyl, Carboxy, C₁-C₆-Alkoxycarbonyl oder Phenyl, das substituiert sein kann durch Nitro, Halogen, C₁-C₂-Alkyl, C₁-C₄-Alkoxy, Trifluormethyl oder Trifluormethoxy, oder
- für Cycloalkyl mit 3 bis 8 Kohlenstoffatomen steht, oder
- für Phenyl steht, das gegebenenfalls substituiert sein kann durch Nitro, Halogen, C₁-C₂-Alkyl oder C₁-C₄-Alkoxy,
und
Y - für einen Rest 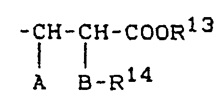 steht, worin
A - für Wasserstoff oder Methyl steht
und
B - für eine Gruppe der Formel -NH, -NH-CO-, -NH-CS-, -NH-COO-, -NH-SO₂- oder -NH-CO-NH- oder -NH-CS-NH- steht,
R¹³
- für geradkettiges oder verzweigtes Alkyl mit bis zu 12 Kohlenstoffatomen steht, das substituiert sein kann durch Halogen, Hydroxy, Carboxy, Cyano, C₁-C₈-Alkoxycarbonyl, Carbonyl, Alkylamino oder Dialkylamino mit bis zu 8 Kohlenstoffatomen, Carbamoyl, C₁-C₆-Alkoxy oder Phenyl, das substituiert sein kann durch Nitro, Cyano, Trifluormethyl, Trifluormethoxy, Halogen, C₁-C₆-Alkoxy oder C₁-C₂-Alkyl, oder
- für Cycloalkyl mit 3 bis 8 Kohlenstoffatomen, oder
- für Phenyl steht, das substituiert sein kann durch Halogen, C₁-C₃-Alkyl, C₁-C₃-Alkoxy, C₁-C₃-Halogenalkyl oder C₁-C₃-Halogenalkoxy,
R¹⁴
- für Wasserstoff steht, oder
- für geradkettiges oder verzweigtes Alkyl oder Alkenyl mit jeweils bis zu 12 Kohlenstoffatomen steht, das substituiert sein kann durch Halogen, Hydroxy, C₁-C₈-Alkoxy, Nitro, Cyano, C₁-C₈-Alkylthio, Carboxy, C₁-C₈-Alkoxycarbonyl oder Phenyl, das substutiert sein kann durch Nitro, Cyano, Trifluormethyl, Trifluormethoxy, C₁-C₈-Alkyl, Halogen oder C₁-C₈-Alkoxy, oder
- für Cycloalkyl mit 3 bis 8 Kohlenstoff-Atomen steht, oder
- für Aryl mit 6 bis 10 Kohlenstoffatomen steht, das bis zu 5-fach gleich oder verschieden substituiert sein kann durch Nitro, Cyano, Halogen, C₁-C₈-Alkyl, C₁-C₈-Alkoxy, C₁-C₈-Alkylthio, Carbamoyl, Dialkylcarbamoyl mit jeweils bis zu 6 Kohlenstoffatomen je Alkylgruppe, Carboxy, C₁-C₈-Alkoxycarbonyl, C₁-C₆-Halogenalkyl, C₁-C₆-Halogenalkoxy, C₁-C₆-Halogenalkylthio, C₁-C₆-Alkylsulfonyl, C₁-C₆-Alkylsulfamoyl, Amino, C₁-C₈-Alkylamino, Di-alkylamino mit jeweils bis zu 8 Kohlenstoffatomen je Alkylgruppe oder C₁-C₈-Acylamino, oder
- für einen 5- bis 7-gliedrigen gesättigten oder ungesättigten, heterocyclischen Ring steht, der als Heteroatom ein bis drei Sauerstoff-, Schwefel- und/oder Stickstoffatome enthalten kann,
D - für Sauerstoff oder für die Gruppe =NH steht und
D*- für Hydroxy oder die Gruppe NH₂ steht, und
E - für Wasserstoff oder im Falle (IIa) für Wasserstoff oder die Gruppe =CHR¹ steht, wobei
R¹ - für geradkettiges oder verzweigtes Alkyl mit bis zu 6 Kohlenstoffatomen steht, das gegebenenfalls substituiert sein kann durch Halogen, Hydroxy, C₁-C₈-Alkoxy, Carboxy, C₁-C₈-Alkoxycarbonyl oder Phenyl, oder
- für Cycloalkyl mit 3 bis 6 Kohlenstoffatomen steht, oder
- für Aryl mit 6 bis 10 Kohlenstoffatomen steht, das bis zu 3-fach gleich oder verschieden substituiert sein kann durch Nitro, Cyano, C₁-C₆-Halogenalkyl, Halogen, C₁-C₆-Alkyl, C₁-C₆-Alkoxycarbonyl oder durch C₁-C₆-Halogenalkoxy, C₁-C₄-Halogenalkylthio, Carbamoyl, Dialkylcarbamoyl mit bis zu 6 Kohlenstoffatomen je Alkylgruppe, C₂-C₈-Alkenyl, das gegebenenfalls substituiert sein kann durch C₁-C₆-Alkoxycarbonyl, oder durch gegebenenfalls durch C₁-C₆-Alkyl, C₁-C₆-Alkoxy, Nitro, Halogen, Cyano, C₁-C₄-Halogenalkyl oder C₁-C₄-Halogenalkoxy substituiertes Phenylsulfonyloxy, oder durch C₁-C₈-Alkylamino, Dialkylamino mit jeweils bis zu 6 Kohlenstoffatomen je Alkylgruppe, C₁-C₈-Acylamino oder durch C₁-C₈-Alkoxy oder C₁-C₈-Alkylthio, die gegebenenfalls substituiert sein können durch Cyclohexyl oder Phenyl, das seinerseits substituiert sein kann durch Halogen, C₁-C₆-Alkyl, C₁-C₆-Alkoxy, Nitro, C₁-C₄-Halogenalkyl, C₁-C₂-Alkoxycarbonyl,Cyano oder C₁-C₄-Halogenalkoxy, oder
- für einen mono-, bi- oder tricyclischen, gesättigten oder ungesättigten Heterocyclus steht, der bis zu 3 Heteroatome aus der Gruppe N, O oder S enthalten kann, der substituiert sein kann durch C₁-C₄-Alkylthio,
mit der Maßgabe, daß Y nicht tert.-Butyl sein kann; wenn R⁴ für Methyl, D für Sauerstoff und E für zwei Wasserstoffatome steht.

2. Verbindungen der allgemeinen Formel (II) gemäß Anspruch 1,
in welcher
R¹ - für geradkettiges oder verzweigtes Alkyl mit bis zu 4 Kohlenstoffatomen steht, das gegebenenfalls substituiert sein kann durch Phenyl, C₁-C₂-Alkoxycarbonyl oder Carboxy, oder - für Phenyl steht, das bis zu 3-fach gleich oder verschieden substituiert sein kann durch Nitro, Cyano, C₁-C₄-Halogenalkyl, Halogen, C₁-C₄-Alkyl, C₁-C₄-Alkoxycarbonyl, C₁-C₂-Halogenalkoxy, C₁-C₂-Halogenalkylthio, C₁-C₄-Alkoxycarbonyl, oder durch gegebenenfalls durch C₁-C₂-Alkyl oder Halogen, substituiertes Phenylsulfonyloxy oder durch C₁-C₄-Alkoxy oder C₁-C₄-Alkylthio, die substituiert sein können durch Cyclohexyl oder Phenyl, das gegebenenfalls substituiert sein kann durch Halogen, C₁-C₄-Alkyl, C₁-C₄-Alkoxy, Nitro, C₁-C₄-Halogenalkyl, Cyano oder C₁-C₄-Halogenalkoxy, oder
- für einen mono- oder bicyclischen ungesättigten Heterocyclus steht, der bis zu 3 Heteroatome aus der Gruppe N, O oder S enthalten kann, der gegebenenfalls substituiert sein kann durch C₁-C₃-Alkylthio
und
R⁴
- für geradkettiges oder verzweigtes Alkyl mit bis zu 6 Kohlenstoffatomen steht, das durch Halogen, Hydroxy, Carboxy, C₁-C₄-Alkoxycarbonyl substituiert sein kann, oder durch Phenyl substituiert sein kann, das seinerseits substituiert sein kann durch Halogen, C₁-C₂-Alkyl, C₁-C₂-Alkoxy, Trifluormethyl oder Trifluormethoxy, oder
- für Cycloalkyl mit 3 bis 6 Kohlenstoffatomen steht, oder
- für Phenyl steht, das gegebenenfalls substituiert sein kann durch Halogen, C₁-C₂-Alkyl oder C₁-C₂-Alkoxy
und
Y - für einen Rest 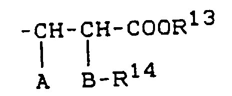 steht, worin
A - für Wasserstoff oder Methyl steht
und
B - für eine Gruppe der Formel -NH, -NH-CO-, -NH-COO-, -NH-SO₂- oder -NH-CO-NH- steht
und
R¹³
- für geradkettiges oder verzweigtes Alkyl mit bis zu 8 Kohlenstoffatomen steht, das substituiert sein kann durch Halogen, Hydroxy, C₁-C₃-Alkoxy, Trifluormethyl, Carboxy, C₁-C₆-Alkoxycarbonyl oder Phenyl, das seinerseits substituiert sein kann durch Trifluormethyl, Trifluormethoxy, Halogen, C₁-C₃-Alkoxy oder C₁-C₂-Alkyl, oder
- für Cycloalkyl mit 3 bis 6 Kohlenstoffatomen steht, oder
- für Phenyl steht, das durch Halogen, C₁-C₂-Alkyl oder C₁-C₂-Alkoxy substituiert sein kann,
und
R¹⁴
- Wasserstoff bedeutet, oder
- für geradkettiges oder verzweigtes Alkyl oder Alkenyl mit jeweils bis zu 8 Kohlenstoffatomen steht, das substituiert sein kann durch Halogen, Hydroxy, C₁-C₄-Alkoxy, Cyano, C₁-C₄-Alkylthio, Carboxy, C₁-C₄-Alkoxycarbonyl oder Phenyl, das seinerseits substituiert sein kann durch Trifluormethyl, Trifluormethoxy, C₁-C₄-Alkyl, Halogen oder C₁-C₄-Alkoxy, oder
- für Cycloalkyl mit 3 bis 6 Kohlenstoffatomen steht, oder
- für Phenyl steht, das bis zu 2-fach gleich oder verschieden substituiert sein kann durch Nitro, Cyano, Halogen, C₁-C₄-Alkyl, C₁-C₄-Alkoxy, C₁-C₄-Alkylthio, Carbamoyl, Dialkylcarbamoyl mit bis zu 4 Kohlenstoffatomen je Alkylgruppe, Carboxy, C₁-C₆-Alkoxycarbonyl, C₁-C₃-Halogenalkyl, C₁-C₃-Halogenalkoxy, C₁-C₃-Halogenalkylthio, C₁-C₃-Alkylsulfonyl, C₁-C₃-Alkylsulfamoyl, Amino, C₁-C₄-Alkylamino, Dialkylamino mit jeweils bis zu 4 Kohlenstoffatomen je Alkylgruppe oder C₁-C₄-Acylamino; oder
- für einen 5- bis 6-gliedrigen ungesättigten heterocyclischen Ring steht, der als Heteroatom ein Stickstoffatom enthalten kann,

3. Verbindungen der allgemeinen Formel (II) gemäß Anspruch 1,
in welcher
R¹
- für geradkettiges oder verzweigtes Alkyl mit bis zu 4 Kohlenstoffatomen steht, oder
- für Cyclopropyl, Cyclopentyl oder Cyclohexyl steht, oder
- für Phenyl steht, das bis zu 2-fach gleich oder verschieden substituiert sein kann durch Nitro, Cyano, Trifluormethyl, Fluor, Chlor, Methyl, Methoxy, Trifluormethoxy, Difluormethoxy, gegebenenfalls durch Methyl, Fluor oder Chlor substituiertes Phenylsulfonyloxy, oder durch C₁-C₄-Alkoxy oder C₁-C₄-Alkylthio, die jeweils substituiert sein können durch Cyclohexyl oder Phenyl, das seinerseits durch Methyl, Fluor, Chlor oder Methoxy substituiert sein kann, oder
- für Pyridyl steht
und
R⁴
- für geradkettiges oder verzweigtes Alkyl mit bis zu 4 Kohlenstoffatomen steht, oder
- für Cyclopropyl steht,
und
Y - für einen Rest 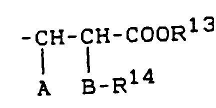 steht, worin
A - für Wasserstoff oder Methyl steht,
und
B - für eine Gruppe der Formel -NH-, -NH-CO-, -NH-COO- oder -NH-SO₂- oder -NH-CO-NH-steht,
und
R¹³ - für geradkettiges oder verzweigtes Alkyl mit bis zu 6 Kohlenstoffatomen steht, das substituiert sein kann durch Hydroxy, Methoxy oder Phenyl,
und
R¹⁴
- Wasserstoff bedeutet, oder
- für geradkettiges oder verzweigtes Alkyl mit bis zu 6 Kohlenstoffatomen steht, das substituiert sein kann durch Hydroxy, C₁-C₂-Alkoxycarbonyl oder durch Phenyl, das seinerseits substituiert sein kann durch Methyl, Trifluormethyl, Fluor oder Chlor, oder
- für Cyclopropyl, Cyclopentyl oder Cyclohexyl steht, oder
- für Phenyl steht, das bis zu 2-fach gleich oder verschieden substituiert sein kann
durch Nitro, Cyano, Fluor, Chlor, C₁-C₂-Alkyl, C₁-C₄-Alkoxy, C₁-C₂-Alkylthio, Carbamoyl, Carboxy, C₁-C₄-Alkoxycarbonyl, Trifluormethyl, Trifluormethoxy, C₁-C₂-Alkylsulfamoyl oder C₁-C₂-Alkylamino, oder
- für Pyridyl steht.

4. Verfahren zur Herstellung von Verbindungen der allgemeinen Formel (II) 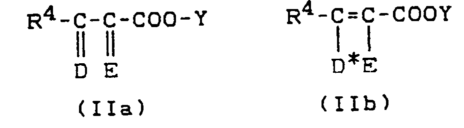 in welcher
R⁴ - für geradkettiges oder verzweigtes Alkyl oder Alkenyl mit bis zu 12 Kohlenstoffatomen steht, das gegebenenfalls substituiert sein kann durch Halogen, Hydroxy, C₁-C₆-Alkoxy, Trifluormethyl, Carboxy, C₁-C₆-Alkoxycarbonyl oder Phenyl, das substituiert sein kann durch Nitro, Halogen, C₁-C₂-Alkyl, C₁-C₄-Alkoxy, Trifluormethyl oder Trifluormethoxy, oder
- für Cycloalkyl mit 3 bis 8 Kohlenstoffatomen steht, oder
- für Phenyl steht, das gegebenenfalls substituiert sein kann durch Nitro, Halogen, C₁-C₂-Alkyl oder C₁-C₄-Alkoxy,
und
Y - für einen Rest 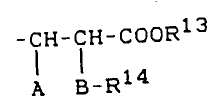 steht, worin
A - für Wasserstoff oder Methyl steht
und
B - für eine Gruppe der Formel -NH, -NH-CO-, -NH-CS-, -NH-COO-, -NH-SO₂- oder -NH-CO-NH- oder -NH-CS-NH- steht,
R¹³
- für geradkettiges oder verzweigtes Alkyl mit bis zu 12 Kohlenstoffatomen steht, das substituiert sein kann durch Halogen, Hydroxy, Carboxy, Cyano, C₁-C₈-Alkoxycarbonyl, Carbonyl, Alkylamino oder Dialkylamino mit bis zu 8 Kohlenstoffatomen, Carbamoyl, C₁-C₆-Alkoxy oder Phenyl, das substituiert sein kann durch Nitro, Cyano, Trifluormethyl, Trifluormethoxy, Halogen, C₁-C₆-Alkoxy oder C₁-C₂-Alkyl, oder
- für Cycloalkyl mit 3 bis 8 Kohlenstoffatomen, oder
- für Phenyl steht, das substituiert sein kann durch Halogen, C₁-C₃-Alkyl, C₁-C₃-Alkoxy, C₁-C₃-Halogenalkyl oder C₁-C₃-Halogenalkoxy,
R¹⁴
- für Wasserstoff steht, oder
- für geradkettiges oder verzweigtes Alkyl oder Alkenyl mit jeweils bis zu 12 Kohlenstoffatomen steht, das substituiert sein kann durch Halogen, Hydroxy, C₁-C₈-Alkoxy, Nitro, Cyano, C₁-C₈-Alkylthio, Carboxy, C₁-C₈-Alkoxycarbonyl oder Phenyl, das substutiert sein kann durch Nitro, Cyano, Trifluormethyl, Trifluormethoxy, C₁-C₈-Alkyl, Halogen oder C₁-C₈-Alkoxy, oder
- für Cycloalkyl mit 3 bis 8 Kohlenstoffatomen steht, oder
- für Aryl mit 6 bis 10 Kohlenstoffatomen steht, das bis zu 5-fach oleich oder verschieden substituiert sein kann durch Nitro, Cyano, Halogen, C₁-C₅-Alkyl, C₁-C₈-Alkoxy, C₁-C₈-Alkylthio, Carbamoyl, Dialkylcarbamoyl mit jeweils bis zu 6 Kohlenstoffatomen je Alkylgruppe, Carboxy, C₁-C₈-Alkoxycarbonyl, C₁-C₆-Halogenalkyl, C₁-C₆-Halogenalkoxy, C₁-C₆-Halogenalkylthio, C₁-C₆-Alkylsulfonyl, C₁-C₆-Alkylsulfamoyl, Amino, C₁-C₈-Alkylamino, Dialkylamino mit jeweils bis zu 8 Kohlenstoffatomen je Alkylgruppe oder C₁-C₈-Acylamino, oder
- für einen 5- bis 7-gliedrigen gesättigten oder ungesättigten, heterocyclischen Ring steht, der als Heteroatom ein bis drei Sauerstoff-, Schwefel- und/oder Stickstoffatome enthalten kann,
D - für Sauerstoff oder für die Gruppe =NH steht
D*- für Hydroxy oder die Gruppe NH₂ steht, und
E - für Wasserstoff oder für den Fall (IIa) für Wasserstoff oder die Gruppe =CHR¹ steht, wobei
R¹
- für geradkettiges oder verzweigtes Alkyl mit bis zu 6 Kohlenstoffatomen steht, das gegebenenfalls substituiert sein kann durch Halogen, Hydroxy, C₁-C₈-Alkoxy, Carboxy, C₁-C₈-Alkoxycarbonyl oder Phenyl, oder
- für Cycloalkyl mit 3 bis 6 Kohlenstoffatomen steht, oder
- für Aryl mit 6 bis 10 Kohlenstoffatomen steht, das bis zu 3-fach gleich oder verschieden substituiert sein kann durch Nitro, Cyano, C₁-C₆-Halogenalkyl, Halogen, C₁-C₆-Alkyl, C₁-C₆-Alkoxycarbonyl oder durch C₁-C₆-Halogenalkoxy, C₁-C₄-Halogenalkylthio, Carbamoyl, Dialkylcarbamoyl mit bis zu 6 Kohlenstoffatomen je Alkylgruppe, C₂-C₈-Alkenyl, das gegebenenfalls substituiert sein kann durch C₁-C₆-Alkoxycarbonyl, oder durch gegebenenfalls durch C₁-C₆-Alkyl, C₁-C₆-Alkoxy, Nitro, Halogen, Cyano, C₁-C₄-Halogenalkyl oder C₁-C₄-Halogenalkoxy substituiertes Phenylsulfonyloxy, oder durch C₁-C₈-Alkylamino, Dialkylamino mit jeweils bis zu 6 Kohlenstoffatomen je Alkylgruppe, C₁-C₈-Acylamino oder durch C₁-C₈-Alkoxy oder C₁-C₈-Alkylthio, die gegebenenfalls substituiert sein können durch Cyclohexyl oder Phenyl, das seinerseits substituiert sein kann durch Halogen, C₁-C₆-Alkyl, C₁-C₆-Alkoxy, Nitro, C₁-C₄-Halogenalkyl, C₁-C₂-Alkoxycarbonyl,Cyano oder C₁-C₄-Halogenalkoxy, oder
- für einen mono-, bi- oder tricyclischen, gesättigten oder ungesättigten Heterocyclus steht, der bis zu 3 Heteroatome aus der Gruppe N, O oder S enthalten kann, der substituiert sein kann durch C₁-C₄-Alkylthio,
mit der Maßgabe, daß Y nicht tert.-Butyl sein kann, wenn R⁴ für Methyl, D für Sauerstoff und E für zwei Wasserstoffatome steht,
dadurch gekennzeichnet, daß man
(A) für die Herstellung von Verbindungen der Formel (II),
in welcher
R⁴ für Methyl steht,
D für Sauerstoff steht,
E für Wasserstoff steht und
Y die oben angegebene Bedeutung hat,
Verbindungen der allgemeinen Formel (VIII)
HO-Y (VIII)
in welcher
Y die oben angegebene Bedeutung hat,
mit Diketen der allgemeinen Formel (IX) 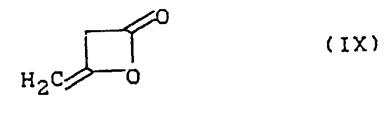 umsetzt,
oder
(B) Verbindungen der Formel (II),
in welcher
R⁴ und Y die oben angegebene Bedeutung haben,
D für Sauerstoff steht und
E für Wasserstoff steht, herstellt,
indem man Verbindungen der Formel (VIII) mit Verbindungen der allgemeinen Formel (X) 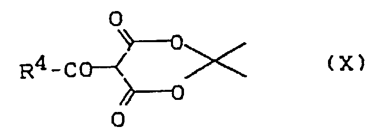 in welcher
R⁴ die oben angegebene Bedeutung hat,
umsetzt,
oder
(C) Verbindungen der allgemeinen Formel (II),
in welcher
R⁴ und Y die oben angegebene Bedeutung haben;
D für -NH steht bzw. D* für NH₂ steht, und
E für Wasserstoff steht;
herstellt,
indem man Verbindungen der allgemeinen Formel (II),
in welcher
D für Sauerstoff steht und
R⁴ und Y die oben angegebene Bedeutung haben und
E für Wasserstoff steht;
mit Ammoniak umsetzt,
oder
(D) Verbindungen der allgemeinen Formel (II),
in welcher
R⁴ und Y die oben angegebene Bedeutung haben,
D Sauerstoff bedeutet und
E für die Gruppe =CH-R¹ steht,
erhält, indem man Aldehyde der allgemeinen Formel (III),
in welcher
R¹ die oben angegebene Bedeutung hat,
mit Verbindungen der allgemeinen Formel (II),
in welcher
D für Sauerstoff und
E für Wasserstoff steht und
R⁴ und Y die oben angegebene Bedeutung haben,
umsetzt.

5. Verwendung von Verbindungen der allgemeinen Formel (II) zur Herstellung von pharmakologisch wirksamen Dihydropyridinen.

## Claims

1. Compounds of the general formulae (II) 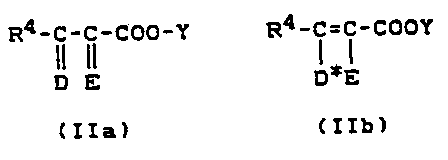 in which
R⁴ represents straight-chain or branched alkyl or alkenyl having up to 12 carbon atoms and which can optionally be substituted by halogen, hydroxyl, C₁-C₆-alkoxy, trifluoromethyl, carboxyl, C₁-C₆-alkoxycarbonyl or phenyl which can be substituted by nitro, halogen, C₁-C₂-alkyl, C₁-C₄-alkoxy, trifluoromethyl or trifluoromethoxy, or
- represents cycloalkyl having 3 to 8 carbon atoms, or
- represents phenyl which can optionally be substituted by nitro, halogen, C₁-C₂-alkyl or C₁-C₄-alkoxy,
and
Y - represents a radical 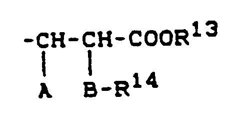 wherein
A - represents hydrogen or methyl
and
B - represents a group of the formula -NH, -NH-CO-, -NH-CS-, -NH-COO-, -NH-SO₂- or -NH-CO-NH- or -NH-CS-NH-,
R¹³
- represents straight-chain or branched alkyl having up to 12 carbon atoms and which can be substituted by halogen, hydroxyl, carboxyl, cyano, C₁-C₈-alkoxycarbonyl, carbonyl, alkylamino or dialkylamino having up to 8 carbon atoms, carbamoyl, C₁-C₆-alkoxy or phenyl which can be substituted by nitro, cyano, trifluoromethyl, trifluoromethoxy, halogen, C₁-C₆-alkoxy or C₁-C₂-alkyl, or
- represents cycloalkyl having 3 to 8 carbon atoms, or
- represents phenyl which can be substituted by halogen, C₁-C₃-alkyl, C₁-C₃-alkoxy, C₁-C₃-halogenoalkyl or C₁-C₃-halogenoalkoxy,
R¹⁴
- represents hydrogen, or
- represents straight-chain or branched alkyl or alkenyl each having up to 12 carbon atoms and which can be substituted by halogen, hydroxyl, C₁-C₈-alkoxy, nitro, cyano, C₁-C₈-alkylthio, carboxyl, C₁-C₈-alkoxycarbonyl or phenyl which can be substituted by nitro cyano, trifluoromethyl, trifluoromethoxy, C₁-C₈-alkyl, halogen or C₁-C₈-alkoxy, or
- represents cycloalkyl having 3 to 8 carbon atoms, or
- represents aryl having 6 to 10 carbon atoms which can be monosubstituted to pentasubstituted by identical or different nitro, cyano, halogen, C₁-C₈-alkyl, C₁-C₈-alkoxy, C₁-C₈-alkylthio, carbamoyl or dialkylcarbamoyl in each case having up to 6 carbon atoms per alkyl group, carboxyl, C₁-C₈-alkoxycarbonyl, C₁-C₆-halogenoalkyl, C₁-C₆-halogenoalkoxy, C₁-C₆-halogenoalkylthio, C₁-C₆-alkylsulphonyl, C₁-C₆-alkylsulphamoyl, amino, C₁-C₈-alkylamino or dialkylamino in each case having up to 8 carbon atoms per alkyl group or C₁-C₈-acylamino, or
- represents a 5- to 7-membered saturated or unsaturated, heterocyclic ring which can contain one to three oxygen, sulphur and/or nitrogen atoms as hetero atoms,
D - represents oxygen or the group =NH and
D*- represents hydroxyl or the group NH₂ and
E - represents hydrogen or in the case of (IIa) hydrogen or the group =CHR¹ where
R¹- represents straight-chain or branched alkyl having up to 6 carbon atoms which can optionally be substituted by halogen, hydroxyl, C₁-C₈-alkoxy, carboxyl, C₁-C₈-alkoxycarbonyl or phenyl, or
- represents cycloalkyl having 3 to 6 carbon atoms, or
- represents aryl having 6 to 10 carbon atoms which can be monosubstituted, disubstituted or trisubstituted by identical or different nitro, cyano, C₁-C₆-halogenoalkyl, halogen, C₁-C₆-alkyl or C₁-C₆-alkoxycarbonyl, or by C₁-C₆-halogenoalkoxy, C₁-C₄-halogenoalkylthio, carbamoyl, dialkylcarbamoyl having up to 6 carbon atoms per alkyl group, or C₂-C₈-alkeny which can optionally be substituted by C₁-C₆-alkoxycarbonyl, or by phenylsulphonyloxy which is optionally substituted by C₁-C₆-alkyl, C₁-C₆-alkoxy, nitro, halogen, cyano, C₁-C₄-halogenoalkyl or C₁-C₄-halogenoalkoxy, or by C₁-C₈-alkylamino or dialkylamino each having up to 6 carbon atoms per alkyl group, or C₁-C₈-acylamino, or by C₁-C₈-alkoxy or C₁-C₈-alkylthio, each of which can optionally be substituted by cyclohexyl or phenyl which, in turn, can be substituted by halogen, C₁-C₆-alkyl, C₁-C₆-alkoxy, nitro, C₁-C₄-halogenoalkyl, C₁-C₂-alkoxycarbonyl, cyano or C₁-C₄-halogenoalkoxy, or
- represents a mono-, bi- or tricyclic, saturated or unsaturated heterocycle which can contain up to 3 hetero atoms from the group N, O or S and which can be substituted by C₁-C₄-alkylthio,
with the proviso that Y cannot be tert-butyl when R⁴ represents methyl, D represents oxygen and E represents two hydrogen atoms.

2. Compounds of the general formula (II) according to Claim 1,
in which
R¹
- represents straight-chain or branched alkyl having up to 4 carbon atoms which can optionally be substituted by phenyl, C₁-C₂-alkoxycarbonyl or carboxyl, or
- represents phenyl which can be monosubstituted, disubstituted or trisubstituted by identical or different nitro, cyano, C₁-C₄-halogenoalkyl, halogen, C₁-C₄-alkyl, C₁-C₄-alkoxycarbonyl, C₁-C₂-halogenoalkoxy, C₁-C₂-halogenoalkylthio or C₁-C₄-alkoxycarbonyl, or by phenylsulphonyloxy which is optionally substituted by C₁-C₂-alkyl or halogen, or by C₁-C₄-alkoxy or C₁-C₄-alkylthio, each of which can be substituted by cyclohexyl or phenyl which can optionally be substituted by halogen, C₁-C₄-alkyl, C₁-C₄-alkoxy, nitro, C₁-C₄-halogenoalkyl, cyano or C₁-C₄-halogenoalkoxy, or
- represents a mono- or bicyclic unsaturated heterocycle which can contain up to 3 hetero atoms from the group N, O or S, and which can optionally be substituted by C₁-C₃-alkylthio,
and
R⁴
- represents straight-chain or branched alkyl having up to 6 carbon atoms which can be substituted by halogen, hydroxyl, carboxyl or C₁-C₄-alkoxycarbonyl, or can be substituted by phenyl which, in turn, can be substituted by halogen, C₁-C₂-alkyl, C₁-C₂-alkoxy, trifluoromethyl or trifluoromethoxy, or
- represents cycloalkyl having 3 to 6 carbon atoms, or
- represents phenyl which can optionally be substituted by halogen, C₁-C₂-alkyl or C₁-C₂-alkoxy
and
Y - represents a radical 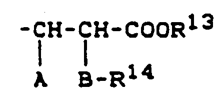 wherein
A - represents hydrogen or methyl
and
B - represents a group of the formula -NH, -NH-CO-, -NH-COO-, -NH-SO₂- or -NH-CO-NH-
and
R¹³
- represents straight-chain or branched alkyl having up to 8 carbon atoms which can be substituted by halogen, hydroxyl, C₁-C₃-alkoxy, trifluoromethyl, carboxyl, C₁-C₆-alkoxycarbonyl or phenyl which, in turn, can be substituted by trifluoromethyl, trifluoromethoxy, halogen, C₁-C₃-alkoxy or C₁-C₂-alkyl, or
- represents cycloalkyl having 3 to 6 carbon atoms, or
- represents phenyl which can be substituted by halogen, C₁-C₂-alkyl or C₁-C₂-alkoxy,
and
R¹⁴
- represents hydrogen, or
- represents straight-chain or branched alkyl or alkenyl each having up to 8 carbon atoms which can be substituted by halogen, hydroxyl, C₁-C₄-alkoxy, cyano, C₁-C₄-alkylthio, carboxyl, C₁-C₄-alkoxycarbonyl or phenyl which, in turn, can be substituted by trifluoromethyl, trifluoromethoxy, C₁-C₄-alkyl, halogen or C₁-C₄-alkoxy, or
- represents cycloalkyl having 3 to 6 carbon atoms, or
- represents phenyl which can be monosubstituted or disubstituted by identical or different nitro, cyano, halogen, C₁-C₄-alkyl, C₁-C₄-alkoxy, C₁-C₄-alkylthio, carbamoyl, dialkylcarbamoyl having up to 4 carbon atoms per alkyl group, carboxyl, C₁-C₆-alkoxycarbonyl, C₁-C₃-halogenoalkyl, C₁-C₃-halogenoalkoxy, C₁-C₃-halogenoalkylthio, C₁-C₃-alkylsulphonyl, C₁-C₃-alkylsulphamoyl, amino, C₁-C₄-alkylamino or dialkylamino in each case having up to 4 carbon atoms per alkyl group or C₁-C₄-acylamino or
- represents a 5- to 6-membered unsaturated heterocyclic ring which can contain a nitrogen atom as a hetero atom.

3. Compounds of the general formula (II) according to Claim 1,
in which
R¹
- represents straight-chain or branched alkyl having up to 4 carbon atoms,
- represents cyclopropyl, cyclopentyl or cyclohexyl, or
- represents phenyl which can be monosubstituted or disubstituted by identical or different nitro, cyano, trifluoromethyl, fluorine, chlorine, methyl, methoxy, trifluoromethoxy, difluoromethoxy or phenylsulphonyloxy which is optionally substituted by methyl, fluorine or chlorine, or by C₁-C₄-alkoxy or C₁-C₄-alkylthio which can each be substituted by cyclohexyl or phenyl which, in turn, can be substituted by methyl, fluorine, chlorine or methoxy, or
- represents pyridyl
and
R⁴
- represents straight-chain or branched alkyl having up to 4 carbon atoms, or
- represents cyclopropyl,
and
Y - represents a radical 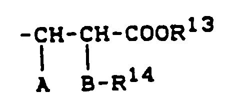 wherein
A - represents hydrogen or methyl,
and
B - represents a group of the formula -NH-, -NH-CO-, -NH-COO-, -NH-SO₂- or -NH-CO-NH-,
and
R¹³ - represents straight-chain or branched alkyl having up to 6 carbon atoms which can be substituted by hydroxyl, methoxy or phenyl,
and
R¹⁴
- denotes hydrogen, or
- represents straight-chain or branched alkyl having up to 6 carbon atoms and which can be substituted by hydroxyl or C₁-C₂-alkoxycarbonyl, or by phenyl which, in turn, can be substituted by methyl, trifluoromethyl, fluorine or chlorine, or
- represents cyclopropyl, cyclopentyl or cyclohexyl, or
- represents phenyl which can be monosubstituted or disubstituted by identical or different nitro, cyano, fluorine, chlorine, C₁-C₂-alkyl, C₁-C₄-alkoxy, C₁-C₂-alkylthio, carbamoyl, carboxyl, C₁-C₄-alkoxycarbonyl, trifluoromethyl, trifluoromethoxy, C₁-C₂-alkylsulphamoyl or C₁-C₂-alkylamino, or
- represents pyridyl.

4. Process for the preparation of compounds of the general formula (II) 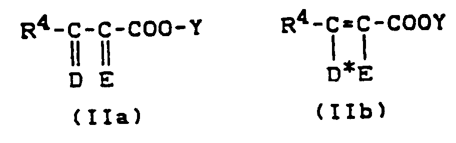 in which
R⁴ represents straight-chain or branched alkyl or alkenyl having up to 12 carbon atoms and which can optionally be substituted by halogen, hydroxyl, C₁-C₆-alkoxy, trifluoromethyl, carboxyl, C₁-C₆-alkoxycarbonyl or phenyl which can be substituted by nitro, halogen, C₁-C₂-alkyl, C₁-C₄-alkoxy, trifluoromethyl or trifluoromethoxy, or
- represents cycloalkyl having 3 to 8 carbon atoms, or
- represents phenyl which can optionally be substituted by nitro, halogen, C₁-C₂-alkyl or C₁-C₄-alkoxy,
and
Y - represents a radical 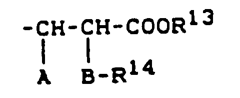 wherein
A - represents hydrogen or methyl
and
B - represents a group of the formula -NH, -NH-CO-, -NH-CS-, -NH-COO-, -NH-SO₂- or -NH-CO-NH- or -NH-CS-NH-,
R¹³
- represents straight-chain or branched alkyl having up to 12 carbon atoms and which can be substituted by halogen, hydroxyl, carboxyl, cyano, C₁-C₈-alkoxycarbonyl, carbonyl, alkylamino or dialkylamino having up to 8 carbon atoms, carbamoyl, C₁-C₆-alkoxy or phenyl which can be substituted by nitro, cyano, trifluoromethyl, trifluoromethoxy, halogen, C₁-C₆-alkoxy or C₁-C₂-alkyl, or
- represents cycloalkyl having 3 to 8 carbon atoms, or
- represents phenyl which can be substituted by halogen, C₁-C₃-alkyl, C₁-C₃-alkoxy, C₁-C₃-halogenoalkyl or C₁-C₃-halogenoalkoxy,
R¹⁴
- represents hydrogen, or
- represents straight-chain or branched alkyl or alkenyl each having up to 12 carbon atoms and which can be substituted by halogen, hydroxyl, C₁-C₈-alkoxy, nitro, cyano, C₁-C₈-alkylthio, carboxyl, C₁-C₈-alkoxycarbonyl or phenyl which can be substituted by nitro, cyano, trifluoromethyl, trifluoromethoxy, C₁-C₈-alkyl, halogen or C₁-C₈-alkoxy, or
- represents cycloalkyl having 3 to 8 carbon atoms, or
- represents aryl having 6 to 10 carbon atoms which can be monosubstituted to pentasubstituted by identical or different nitro, cyano, halogen, C₁-C₈-alkyl, C₁-C₈-alkoxy, C₁-C₈-alkylthio, carbamoyl or dialkylcarbamoyl in each case having up to 6 carbon atoms per alkyl group, carboxyl, C₁-C₈-alkoxycarbonyl, C₁-C₆-halogenoalkyl, C₁-C₆-halogenoalkoxy, C₁-C₆-halogenoalkylthio, C₁-C₆-alkylsulphonyl, C₁-C₆-alkylsulphamoyl, amino, C₁-C₈-alkylamino or dialkylamino in each case having up to 8 carbon atoms per alkyl group or C₁-C₈-acylamino, or
- represents a 5- to 7-membered saturated or unsaturated, heterocyclic ring which can contain one to three oxygen, sulphur and/or nitrogen atoms as hetero atoms,
D - represents oxygen or the group =NH and
D*- represents hydroxyl or the group NH₂, and
E - represents hydrogen or in the case of (IIa) hydrogen or the group =CHR¹ where
R¹
- represents straight-chain or branched alkyl having up to 6 carbon atoms which can optionally be substituted by halogen, hydroxyl, C₁-C₈-alkoxy, carboxyl, C₁-C₈-alkoxycarbonyl or phenyl, or
- represents cycloalkyl having 3 to 6 carbon atoms, or
- represents aryl having 6 to 10 carbon atoms which can be monosubstituted, disubstituted or trisubstituted by identical or different nitro, cyano, C₁-C₆-halogenoalkyl, halogen, C₁-C₆-alkyl or C₁-C₆-alkoxycarbonyl, or by C₁-C₆-halogenoalkoxy C₁-C₄-halogenoalkylthio, carbamoyl, dialkylcarbamoyl having up to 6 carbon atoms per alkyl group, or C₂-C₈-alkenyl which can optionally be substituted by C₁-C₆-alkoxycarbonyl, or by phenylsulphonyloxy which is optionally substituted by C₁-C₆-alkyl, C₁-C₆-alkoxy, nitro, halogen, cyano, C₁-C₄-halogenoalkyl or C₁-C₄-halogenoalkoxy, or by C₁-C₈-alkylamino or dialkylamino each having up to 6 carbon atoms per alkyl group, or C₁-C₈-acylamino, or by C₁-C₈-alkoxy or C₁-C₈-alkylthio, each of which can optionally be substituted by cyclohexyl or phenyl which, in turn, can be substituted by halogen, C₁-C₆-alkyl, C₁-C₆-alkoxy, nitro, C₁-C₄-halogenoalkyl, C₁-C₂-alkoxycarbonyl, cyano or C₁-C₄-halogenoalkoxy, or
- represents a mono-, bi- or tricyclic, saturated or unsaturated heterocycle which can contain up to 3 hetero atoms from the group N, O or S and which can be substituted by C₁-C₄-alkylthio,
with the proviso that Y cannot be tert-butyl when R⁴ represents methyl, D represents oxygen and E represents two hydrogen atoms,
characterized in that
(A) for the preparation of compounds of the formula (II)
in which
R⁴ represents methyl,
D represents oxygen,
E represents hydrogen and
Y has the abovementioned meaning,
compounds of the general formula (VIII)
HO-Y (VIII)
in which
Y has the abovementioned meaning,
are reacted with diketene of the general formula (IX) 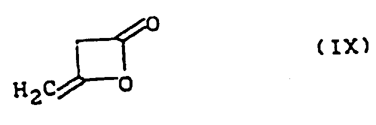 or
(B) compounds of the formula (II)
in which
R⁴ and Y have the abovementioned meaning,
D represents oxygen and
E represents hydrogen,
are prepared by reacting compounds of the formula (VIII) with compounds of the general formula (X) 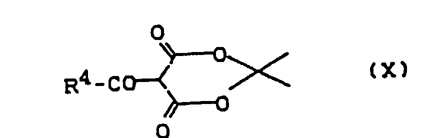 in which
R⁴ has the abovementioned meaning,
or
(C) compounds of the general formula (II)
in which
R⁴ and Y have the abovementioned meaning,
D represents -NH and D* represents NH₂ and
E represents hydrogen,
are prepared by reacting compounds of the general formula (II)
in which
D represents oxygen and
R⁴ and Y have the abovementioned meaning and
E represents hydrogen,
with ammonia,
or
(D) compounds of the general formula (II)
in which
R⁴ and Y have the abovementioned meaning,
D denotes oxygen and
E represents the group =CH-R¹,
are obtained by reacting aldehydes of the general formula (III),
in which
R¹ has the abovementioned meaning,
with compounds of the general formula (II)
in which
D represents oxygen and
E represents hydrogen and
R⁴ and Y have the abovementioned meaning.

5. Use of compounds of the general formula (II) for the preparation of pharmacologically active dihydropyridines.

## Revendications

1. Composés de formule générale (II) 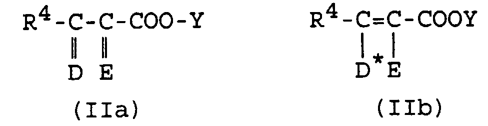 dans laquelle
R⁴ est un groupe alkyle ou alcényle linéaire ou ramifié ayant jusqu'à 12 atomes de carbone, qui peut être substitué le cas échéant par un halogène, un radical hydroxy, alkoxy en C₁ à C₆, trifluorométhyle, carboxy, (alkoxy en C₁ à C₆)-carbonyle ou phényle qui peut être substitué par un radical nitro, halogéno, alkyle en C₁ ou C₂, alkoxy en C₁ à C₄, trifluorométhyle ou trifluorométhoxy, ou bien
- un groupe cycloalkyle ayant 3 à 8 atomes de carbone, ou bien
- un groupe phényle qui peut être substitué le cas échéant par un radical nitro, halogéno, alkyle en C₁ ou C₂ ou alkoxy en C₁ à C₄,
Y - représente un reste 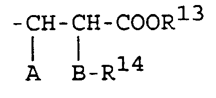 dans lequel
A - représente de l'hydrogène ou un groupe méthyle,
et
B - représente un groupe de formule -NH, -NH-CO-, -NH-CS-, -NH-COO-, -NH-SO₂-
ou -NH-CO-NH- ou -NH-CS-NH-,
R¹³
- représente
- un groupe alkyle linéaire ou ramifié ayant jusqu'à 12 atomes de carbone, qui peut être substitué par un radical halogéno, hydroxy, carboxy, cyano, (alkoxy en C₁ à C₈)-carbonyle, carbonyle alkylamino ou dialkylamino ayant jusqu'à 8 atomes de carbone, carbamoyle, alkoxy en C₁ à C₆ ou phényle, qui peut être substitué par un radical nitro, cyano, trifluorométhyle, trifluorométhoxy, halogéno, alkoxy en C₁ à C₆ ou alkyle en C₁ ou C₂,
- un groupe cycloalkyle ayant 3 à 8 atomes de carbone, ou bien
- un groupe phényle qui peut être substitué par un halogène, un radical alkyle en C₁ à C₃, alkoxy en C₁ à C₃, halogénalkyle en C₁ à C₃, ou halogénalkoxy en C₁ à C₃,
R¹⁴
- représente de l'hydrogène ou
- un groupe alkyle ou un groupe alcényle linéaire ou ramifié ayant chacun jusqu'à 12 atomes de carbone, qui peut être substitué par un radical halogéno, hydroxy, alkoxy en C₁ à C₈, nitro, cyano, alkylthio en C₁ à C₈, carboxy, (alkoxy en C₁ à C₈)-carbonyle ou phényle, qui peut être substitué par un radical nitro, cyano, trifluorométhyle, trifluorométhoxy, alkyle en C₁ à C₈, halogéno ou alkoxy en C₁ à C₈, ou bien
- un groupe cycloalkyle ayant 3 à 8 atomes de carbone, ou bien
- un groupe aryle ayant 6 à 10 atomes de carbone, qui peut être substitué jusqu'à 5 fois identiques ou différentes par un radical nitro, cyano, halogéno, alkyle en C₁ à C₈, alkoxy en C₁ à C₈, alkylthio en C₁ à C₈, carbamoyle, dialkylcarbamoyle ayant dans chaque cas jusqu'à 6 atomes de carbone par groupe alkyle, carboxy, (alkoxy en C₁ à C₈)-carbonyle, halogénalkyle en C₁ à C₆, halogénalkoxy en C₁ à C₆, halogénalkylthio en C₁ à C₆, alkylsulfonyle en C₁ à C₆, alkylsulfamoyle en C₁ à C₆, amino, alkylamino en C₁ à C₈, dialkylamino ayant dans chaque cas jusqu'à 8 atomes de carbone par groupe alkyle, ou acylamino en C₁ à C₈, ou bien
- un noyau hétérocyclique pentagonal à heptagonal saturé ou non saturé, qui peut contenir comme hétéro-atome 1 à 3 atomes d'oxygène, de soufre et/ou d'azote,
D - représente de l'oxygène ou le groupe =NH et
D* - est un groupe hydroxy ou le groupe NH₂, et
E - représente de l'hydrogène ou, dans le cas (IIa), de l'hydrogène ou le groupe =CHR¹, où
R¹
- est un groupe alkyle linéaire ou ramifié ayant jusqu'à 6 atomes de carbone, qui peut être substitué le cas échéant par un radical halogéno, hydroxy, alkoxy en C₁ à C₈, carboxy, (alkoxy en C₁ à C₈)-carbonyle ou phényle, ou bien
- un groupe cycloalkyle ayant 3 à 6 atomes de carbone, ou bien
- un groupe aryle de 6 à 10 atomes de carbone, qui peut être substitué jusqu'à trois fois identiques ou différentes par un radical nitro, cyano, halogénalkyle en C₁ à C₆, halogéno, alkyle en C₁ à C₆, (alkoxy en C₁ à C₆)-carbonyle ou par un radical halogénalkoxy en C₁ à C₆, halogénalkylthio en C₁ à C₄, carbamoyle, dialkylcarbamoyle ayant jusqu'à 6 atomes de carbone par groupe alkyle, alcényle en C₂ à C₈ qui peut être substitué le cas échéant par un radical (alkoxy en C₁ à C₆)-carbonyle, ou par un groupe phénylsulfonyloxy éventuellement substitué par un radical alkyle en C₁ à C₆, alkoxy en C₁ à C₆, nitro, halogéno, cyano, halogénalkyle en C₁ à C₄ ou halogénalkoxy en C₁ à C₄, ou par un groupe alkylamino en C₁ à C₈, un groupe dialkylamino ayant dans chaque cas jusqu'à 6 atomes de carbone par groupe alkyle, un groupe acylamino en C₁ à C₈ ou par un groupe alkoxy en C₁ à C₈ ou un groupe alkylthio en C₁ à C₈ qui peuvent être substitués le cas échéant par un radical cyclohexyle ou par un radical phényle qui peut être substitué de son côté par un radical halogéno, alkyle en C₁ à C₆, alkoxy en C₁ à C₆, nitro, halogénalkyle en C₁ à C₄, (alkoxy en C₁ ou C₂)-carbonyle, cyano ou halogénalkoxy en C₁ à C₄, ou bien
- un hétérocycle monocyclique, bicyclique ou tricyclique saturé ou non saturé, pouvant contenir jusqu'à 3 hétéro-atomes du groupe N, O ou S, qui peut être substitué par un radical alkylthio en C₁ à C₄,
sous réserve que Y ne puisse pas être un groupe tertiobutyle lorsque R⁴ est un groupe méthyle, D est de l'oxygène et E représente deux atomes d'hydrogène.

2. Composés de formule générale (II) suivant la revendication 1,
dans laquelle
R¹
- est un groupe alkyle linéaire ou ramifié ayant jusqu'à 4 atomes de carbone, qui peut être substitué le cas échéant par un radical phényle, (alkoxy en C₁ ou C₂)-carbonyle ou carboxy, ou bien
- un groupe phényle qui peut être substitué jusqu'à trois fois identiques ou différentes par un radical nitro, cyano, halogénalkyle en C₁ à C₄, halogéno, alkyle en C₁ à C₄, (alkoxy en C₁ à C₄)-carbonyle, halogénalkoxy en C₁ ou C₂, halogénalkylthio en C₁ ou C₂, (alkoxy en C₁ à C₄)-carbonyle, ou par un groupe phénylsulfonyloxy éventuellement substitué par un radical alkyle en C₁ ou C₂ ou halogéno, ou par un groupe alkoxy en C₁ à C₄ ou un groupe alkylthio en C₁ à C₄, qui peuvent être substitués par un radical cyclohexyle ou par un radical phényle qui peut être substitué le cas échéant par un radical halogéno, alkyle en C₁ à C₄, alkoxy en C₁ à C₄, nitro, halogénalkyle en C₁ à C₄, cyano ou halogénalkoxy en C₁ à C₄,
ou bien
- un hétérocycle monocyclique ou bicyclique non saturé, contenant jusqu'à 3 hétéro-atomes du groupe N, O ou S, qui peut être substitué le cas échéant par un radical alkylthio en C₁ à C₃,
et
R⁴ est un groupe alkyle linéaire ou ramifié ayant jusqu'à 6 atomes de carbone, qui peut être substitué le cas échéant par un radical halogéno, hydroxy, carboxy, (alkoxy en C₁ à C₄)-carbonyle, ou par un radical phényle qui peut être substitué de son côté par un radical halogéno, alkyle en C₁ ou C₂, alkoxy en C₁ ou C₂, trifluorométhyle ou trifluorométhoxy, ou bien
- un groupe cycloalkyle ayant 3 à 6 atomes de carbone, ou bien
- un groupe phényle qui peut être substitué le cas échéant par un radical halogéno, alkyle en C₁ ou C₂ ou alkoxy en C₁ ou C₂,
et
Y - représente un reste 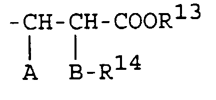 dans lequel
A - est de l'hydrogène ou un groupe méthyle,
et
B - est un groupe de formule -NH, -NH-CO-, -NH-COO-, -NH-SO₂- ou -NH-CO-NH-,
et
R¹³
- représente
- un groupe alkyle linéaire ou ramifié ayant jusqu'à 8 atomes de carbone, qui peut être substitué par un radical halogéno, hydroxy, alkoxy en C₁ à C₃, trifluorométhyle, carboxy, alkoxy en C₁ à C₆)-carbonyle ou par un radical phényle qui peut être substitué de son côté par un radical trifluorométhyle, trifluorométhoxy, halogéno, alkoxy en C₁ à C₃ ou alkyle en C₁ ou C₂, ou bien
- un groupe cycloalkyle ayant 3 à 6 atomes de carbone, ou bien
- un groupe phényle qui peut être substitué par un radical halogéno, alkyle en C₁ ou C₂ ou alkoxy en C₁ ou C₂, et
R¹⁴
- désigne de l'hydrogène ou
- un groupe alkyle ou un groupe alcényle linéaire ou ramifié ayant chacun jusqu'à 8 atomes de carbone, qui peuvent être substitués par un radical halogéno, hydroxy, alkoxy en C₁ à C₄, nitro, cyano, alkylthio en C₁ à C₄, carboxy, (alkoxy en C₁ à C₄)-carbonyle ou par un radical phényle qui peut être substitué de son côté par un radical trifluorométhyle, trifluorométhoxy, alkyle en C₁ à C₄, halogéno ou alkoxy en C₁ à C₄, ou bien
- un groupe cycloalkyle de 3 à 6 atomes de carbone, ou bien
- un groupe phényle qui peut être substitué jusqu'à 2 fois identiques ou différentes par un radical nitro, cyano, halogéno, alkyle en C₁ à C₄, alkoxy en C₁ à C₄, alkylthio en C₁ à C₄, carbamoyle, dialkylcarbamoyle ayant jusqu'à 4 atomes de carbone dans chaque groupe alkyle, carboxy, (alkoxy en C₁ à C₆)-carbonyle, halogénalkyle en C₁ à C₃, halogénalkoxy en C₁ à C₃, halogénalkylthio en C₁ à C₃, alkylsulfonyle en C₁ à C₃, alkylsulfamoyle en C₁ à C₃, amino, alkylamino en C₁ à C₄, dialkylamino ayant dans chaque cas jusqu'à 4 atomes de carbone par groupe alkyle, ou acylamino en C₁ à C₄, ou bien
- un noyau hétérocyclique pentagonal ou hexagonal non saturé, qui peut contenir un atome d'azote comme hétéro-atome.

3. Composés de formule générale (II) suivant la revendication 1,
dans laquelle
R¹
- est un groupe alkyle linéaire ou ramifié ayant jusqu'à 4 atomes de carbone ou
- un groupe cyclopropyle, cyclopentyle ou cyclohexyle, ou bien
- un groupe phényle qui peut être substitué jusqu'à deux fois identiques ou différentes par un radical nitro, cyano, trifluorométhyle, fluoro, chloro, méthyle, méthoxy, trifluorométhoxy, difluorométhoxy, phénylsulfonyloxy portant éventuellement un substituant méthyle, fluoro ou chloro, ou par un radical alkoxy en C₁ à C₄ ou un radical alkylthio en C₁ à C₄ qui peuvent être substitués chacun par un radical cyclohexyle ou un radical phényle qui peut être substitué quant à lui par un radical méthyle, fluoro, chloro ou méthoxy,
ou bien
- un groupe pyridyle,
et
R⁴ est un groupe alkyle linéaire ou ramifié ayant jusqu'à 4 atomes de carbone, ou bien
- un groupe cyclopropyle,
et
Y - représente un reste 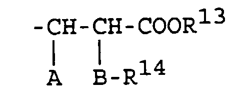 dans lequel
A - est de l'hydrogène ou un groupe méthyle,
et
B - est un groupe de formule -NH, -NH-CO-, -NH-COO- ou -NH-SO₂- ou -NH-CO-NH-,
et
R¹³ - est un groupe alkyle linéaire ou ramifié ayant jusqu'à 6 atomes de carbone, qui peut être substitué par un radical hydroxy, méthoxy ou phényle,
et
R¹⁴
- représente de l'hydrogène,
ou bien
- un groupe alkyle linéaire ou ramifié ayant chacun jusqu'à 6 atomes de carbone, qui peut être substitué par un radical hydroxy, (alkoxy en C₁ ou C₂)-carbonyle ou par un radical phényle qui peut être substitué de son côté par un radical méthyle, trifluorométhyle, fluoro ou chloro,
- un groupe cyclopropyle, cyclopentyle ou cyclohexyle, ou bien
- un groupe phényle qui peut être substitué jusqu'à 2 fois identiques ou différentes par un radical nitro, cyano, fluoro, chloro, alkyle en C₁ ou C₂, alkoxy en C₁ à C₄, alkylthio en C₁ ou C₂, carbamoyle, carboxy, (alkoxy en C₁ à C₄)-carbonyle, trifluorométhyle, trifluorométhoxy, (alkyle en C₁ ou C₂)-sulfamoyle ou alkylamino en C₁ ou C₂, ou bien
- un groupe pyridyle.

4. Procédé de production de composés de formule générale (II) 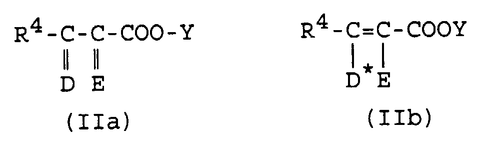 dans laquelle
R⁴ est un groupe alkyle ou alcényle linéaire ou ramifié ayant jusqu'à 12 atomes de carbone, qui peut être substitué le cas échéant par un halogène, un radical hydroxy, alkoxy en C₁ à C₆, trifluorométhyle, carboxy, (alkoxy en C₁ à C₆)-carbonyle ou phényle qui peut être substitué par un radical nitro, halogéno, alkyle en C₁ ou C₂, alkoxy en C₁ à C₄, trifluorométhyle ou trifluorométhoxy, ou bien
- un groupe cycloalkyle ayant 3 à 8 atomes de carbone, ou bien
- un groupe phényle qui peut être substitué le cas échéant par un radical nitro, halogéno, alkyle en C₁ ou C₂ ou alkoxy en C₁ à C₄,
et
Y - représente un reste 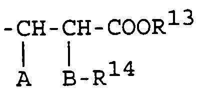 dans lequel
A - représente de l'hydrogène ou un groupe méthyle,
et
B - représente un groupe de formule -NH, -NH-CO-, -NH-CS-, -NH-COO-, -NH-SO₂-
ou -NH-CO-NH- ou -NH-CS-NH-,
R¹³
- représente
- un groupe alkyle linéaire ou ramifié ayant jusqu'à 12 atomes de carbone, qui peut être substitué par un radical halogéno, hydroxy, carboxy, cyano, (alkoxy en C₁ à C₈)-carbonyle, carbonyle, alkylamino ou dialkylamino ayant jusqu'à 8 atomes de carbone, carbamoyle, alkoxy en C₁ à C₆ ou phényle, qui peut être substitué par un radical nitro, cyano, trifluorométhyle, trifluorométhoxy, halogéno, alkoxy en C₁ à C₆ ou alkyle en C₁ ou C₂,
- un groupe cycloalkyle ayant 3 à 8 atomes de carbone, ou bien
- un groupe phényle qui peut être substitué par un halogène, un radical alkyle en C₁ à C₃, alkoxy en C₁ à C₃, halogénalkyle en C₁ à C₃, ou halogénalkoxy en C₁ à C₃,
R¹⁴
- représente de l'hydrogène ou
- un groupe alkyle ou un groupe alcényle linéaire ou ramifié ayant chacun jusqu'à 12 atomes de carbone, qui peut être substitué par un radical halogéno, hydroxy, alkoxy en C₁ à C₈, nitro, cyano, alkylthio en C₁ à C₈, carboxy, (alkoxy en C₁ à C₈)-carbonyle ou phényle, qui peut être substitué par un radical nitro, cyano, trifluorométhyle, trifluorométhoxy, alkyle en C₁ à C₈, halogéno ou alkoxy en C₁ à C₈, ou bien
- un groupe cycloalkyle ayant 3 à 8 atomes de carbone, ou bien
- un groupe aryle ayant 6 à 10 atomes de carbone, qui peut être substitué jusqu'à 5 fois identiques ou différentes par un radical nitro, cyano, halogéno, alkyle en C₁ à C₈, alkoxy en C₁ à C₈, alkylthio en C₁ à C₈, carbamoyle, dialkylcarbamoyle ayant dans chaque cas jusqu'à 6 atomes de carbone par groupe alkyle, carboxy, (alkoxy en C₁ à C₈)-carbonyle, halogénalkyle en C₁ à C₆, halogénalkoxy en C₁ à C₆, halogénalkylthio en C₁ à C₆, alkylsulfonyle en C₁ à C₆, alkylsulfamoyle en C₁ à C₆, amino, alkylamino en C₁ à C₈, dialkylamino ayant dans chaque cas jusqu'à 8 atomes de carbone par groupe alkyle, ou acylamino en C₁ à C₈, ou bien
- un noyau hétérocyclique pentagonal à heptagonal saturé ou non saturé, qui peut contenir comme hétéro-atome 1 à 3 atomes d'oxygène, de soufre et/ou d'azote,
D - représente de l'oxygène ou le groupe =NH et
D* - est un groupe hydroxy ou le groupe NH₂, et
E - représente de l'hydrogène ou, dans le cas (IIa), de l'hydrogène ou le groupe =CHR¹, où
R¹ - est un groupe alkyle linéaire ou ramifié ayant jusqu'à 6 atomes de carbone, qui peut être substitué le cas échéant par un radical halogéno, hydroxy, alkoxy en C₁ à C₈, carboxy, (alkoxy en C₁ à C₈)-carbonyle ou phényle, ou bien
- un groupe cycloalkyle ayant 3 à 6 atomes de carbone, ou bien
- un groupe aryle de 6 à 10 atomes de carbone, qui peut être substitué jusqu'à trois fois identiques ou différentes par un radical nitro, cyano, halogénalkyle en C₁ à C₆, halogéno, alkyle en C₁ à C₆, (alkoxy en C₁ à C₆)-carbonyre ou par un radical halogénalkoxy en C₁ à C₆, halogénalkylthio en C₁ à C₄, carbamoyle, dialkylcarbamoyle ayant jusqu'à 6 atomes de carbone par groupe alkyle, alcényle en C₂ à C₈ qui peut être substitué le cas échéant par un radical (alkoxy en C₁ à C₆)-carbonyreo ou par un groupe phénylsulfonyloxy éventuellement substitué par un radical alkyle en C₁ à C₆, alkoxy en C₁ à C₆, nitro, halogéno, cyano, halogénalkyle en C₁ à C₄ ou halogénalkoxy en C₁ à C₄, ou par un groupe alkylamino en C₁ à C₈, un groupe dialkylamino ayant dans chaque cas jusqu'à 6 atomes de carbone par groupe alkyle, un groupe acylamino en C₁ à C₈ ou par un groupe alkoxy en C₁ à C₈ ou un groupe alkylthio en C₁ à C₈ qui peuvent être substitués le cas échéant par un radical cyclohexyle ou par un radical phényle qui peut être substitué de son côté par un radical halogéno, alkyle en C₁ à C₆, alkoxy en C₁ à C₆, nitro, halogénalkyle en C₁ à C₄, (alkoxy en C₁ ou C₂)-carbonyle, cyano ou halogénalkoxy en C₁ à C₄, ou bien
- un hétérocycle monocyclique, bicyclique ou tricyclique saturé ou non saturé, pouvant contenir jusqu'à 3 hétéro-atomes du groupe N, O ou S, qui peut être substitué par un radical alkylthio en C₁ à C₄,
sous réserve que Y ne puisse pas être un groupe tertiobutyle lorsque R⁴ est un groupe méthyle, D est de l'oxygène et E représente deux atomes d'hydrogène,
caractérisé en ce que
(A) pour la production de composés de formule (II), dans laquelle,
R⁴ est un groupe méthyle,
D est de l'oxygène,
E est de l'hydrogène, et
Y a la définition indiquée ci-dessus,
on fait réagir des composés de formule générale (VIII)
HO-Y (VIII)
dans laquelle
Y a la définition indiquée ci-dessus,
avec le dicétène de formule générale (IX) 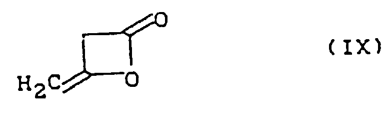 ou bien,
(B) on produit des composés de formule (II), dans laquelle
R⁴ et Y ont la définition indiquée ci-dessus,
D est de l'oxygène, et
E est de l'hydrogène,
en faisant réagir des composés de formule (VIII) avec des composés de formule générale (X) 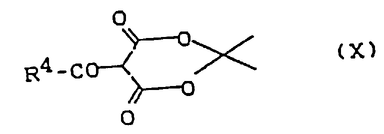 dans laquelle
R⁴ a la définition indiquée ci-dessus,
ou bien
(C) on produit des composés de formule générale (II) dans laquelle
R⁴ et Y ont la définition indiquée ci-dessus,
D est un groupe -NH ou D* est un groupe NH₂ et
E est de l'hydrogène,
en faisant réagir des composés de formule générale (II), dans laquelle
D est de l'oxygène et
R⁴ et Y ont la définition indiquée ci-dessus, et
E est de l'hydrogène,
avec de l'ammoniac,
ou bien
(D) on obtient des composés de formule générale (II), dans laquelle
R⁴ et Y ont la définition indiquée ci-dessus,
D désigne de l'oxygène et
E est le groupe =CH-R¹,
en faisant réagir des aldéhydes de formule générale (III)
dans laquelle
R¹ a la définition indiquée ci-dessus,
avec des composés de formule générale (II) dans laquelle
D est de l'oxygène, et
E est de l'hydrogène et
R⁴ et Y ont la définition indiquée ci-dessus.

5. Utilisation de composés de formule générale (II) pour la production de dihydropyridines douées d'activité pharmacologique.
